(19) Europäisches Patentamt
European Patent Office
Office européen des brevets

(11) EP 2 184 350 B1

(12) EUROPEAN PATENT SPECIFICATION

(45) Date of publication and mention of the grant of the patent:
**03.09.2014 Bulletin 2014/36**

(51) Int Cl.:
***C12N 9/00*** *(2006.01)* ***C12N 9/28*** *(2006.01)*
***C12N 9/42*** *(2006.01)*

(21) Application number: **09014733.1**

(22) Date of filing: **14.12.2000**

(54) **Cellulases having improved thermostability**

Cellulasen mit verbesserter Thermostabilität

Enzymes cellulolytiques dotés de thermostabilité améliorée

(84) Designated Contracting States:
**AT BE CH CY DE DK ES FI FR GB GR IE IT LI LU MC NL PT SE TR**

(30) Priority: **23.12.1999 US 470832**

(43) Date of publication of application:
**12.05.2010 Bulletin 2010/19**

(62) Document number(s) of the earlier application(s) in accordance with Art. 76 EPC:
**00984363.2 / 1 240 524**

(73) Proprietor: **Danisco US Inc.**
**Palo Alto, California 94304 (US)**

(72) Inventors:
- **Day, Anthony G.**
  **San Francisco, CA 94127 (US)**
- **Mitchinson, Colin**
  **Half Moon Bay, CA 94019 (US)**
- **Shaw, Andrew**
  **San Francisco, CA 24109 (US)**

(74) Representative: **Casley, Christopher Stuart et al**
**Mewburn Ellis LLP**
**33 Gutter Lane**
**London**
**EC2V 8AS (GB)**

(56) References cited:
**WO-A-95/24471** **WO-A2-00/14206**
**WO-A2-99/31255**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 184 350 B1

**Description**

## FIELD OF THE INVENTION

The present invention is directed to improved proteins, and particularly enzymes, having altered stability characteristics through the recruitment of residues from a homolog or related protein and to a powerful method which enables the development of such improved proteins with great accuracy.

## BACKGROUND OF THE INVENTION

Methods of improving functional characteristics of proteins have been practiced extensively for the purpose of increasing the usefulness of the proteins in various applications. Such techniques often involve the use of protein engineering and site-specific mutagenesis to isolate and select specific amino acid residues which appear, based on the specific amino acid properties and/or the structure of the protein, to be especially relevant or are shown to be relevant through experimental evidence. For example, Estell et al., U.S. Patent No. 4,760,025 suggest modification of oxidatively unstable enzymes to confer increased stability by altering residues which are particularly susceptible to oxidation, i.e., methionine, lysine, tryptophan and cysteine, among others. Koths et al., U.S. Patent No. 4,752,585, suggest a method of improving the oxidation stability of a therapeutic protein by making a conservative amino acid substitution for each methionyl residue susceptible to chloramine T oxidation. Barr et al., U.S. Patent No. 4,732,973, suggest substituting the active site methionine from human $\alpha_1$-antitrypsin with an oxidatively stable amino acid.

Additional techniques which have been suggested include homology modeling of proteins against evolutionarily close proteins to provide a basis for protein engineering. Siezen et al., Protein Engineering, vol. 4, no. 7, pp. 719-737 (1991) disclose a strategy for improving the properties of proteases used in industrial processes by incorporating or duplicating features from more stable enzymes into a target. Moreover, these techniques require that a attributes from a more desirable protein be imparted to a less desirable protein. In practice, however, the more desirable protein is the target for which it is desired to develop the improved characteristics. Thus, it is not feasible to align such a protein with a more desirable one. This leaves the researcher to look to non-knowledge based techniques for improvement.

Non-knowledge based mutation strategies become necessary where there is a lack of information about the protein of interest or where knowledge based techniques fail. Typical non-knowledge based techniques comprise well known methods including random mutagenesis, error-prone PCR and PCR template switching techniques as well as more recent developments such as shuffling described in Stemmer et al., U.S. Patent No. 5,605,793. Random mutagenesis has been used for many years with various levels of success reported throughout the literature. However, random mutagenesis is a hit or miss strategy due to the immense numbers of potential mutants. For example, a 300 amino acid protein has a potential number of random mutants on the order of $300^{20}$. Preparing, expressing and screening such a great number of mutants, even using present day mass screening techniques, is impossible. Directed evolution techniques such as "gene shuffling" as described by Stemmer or other molecular breeding techniques are limited by the available pool of mutants. However, the pool of mutants must be produced either from nature or using random mutagenesis if knowledge regarding the protein is insufficient. This pool of variants, however, potentially fails to provide an optimally focused pool of diverse organisms necessary for a reasonable degree of success.

There has been a considerable effort in the field of protein engineering to produce more stable enzymes. Nonetheless, given the immense information content present in many important proteins, improved methods for intelligently focusing a pool of mutant molecules toward a specific effect must be developed to fully exploit the range of biological diversity. As disclosed below, the present inventors have developed a novel knowledge based mutagenesis technique which provides for excellent probabilities in terms of producing novel proteins which have improved performance, i.e., functional characteristics. By practicing the present technique, it is possible for a researcher to develop a focused pool of mutants from which the selection of a desirable "winner" protein is facilitated.

WO 00/14206 A2 describes *T. reesei* mutants having substitutions at, *inter alia,* positions 55 and 204 of the numbering used therein.

WO 99/31255 A2 describes *T. reesei* EGIII like cellulases comprising defined amino acid strings.

WO 95/24471 A1 describes alkaline cellulases exhibiting enhanced enzyme activity in the alkaline pH range.

Summary of the invention

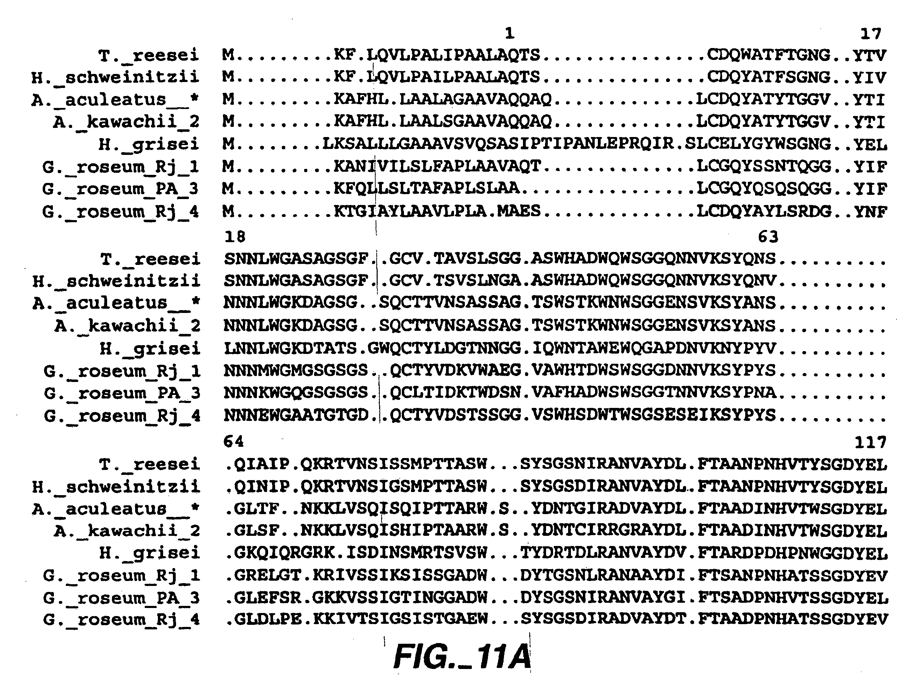

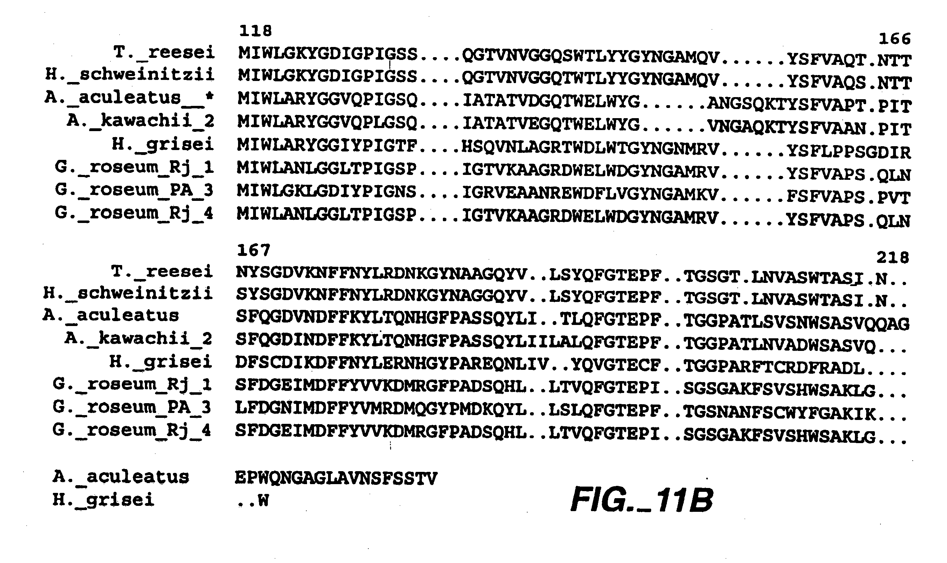

In a first aspect the present invention provides an improved cellulase comprising an amino acid sequence which has been modified from a precursor amino acid sequence having the sequence of *T. reesei* EGIII cellulase shown in Fig. 11 by substitution of one or more amino acid residues, said substitution being selected from: T11S/T16I, G41A, N91D, S143T, T163S, N167S, V210C, and T145E/Y147W, said amino acid numbering being as in Figure 11; wherein the improved cellulase has increased thermostability compared to a cellulase corresponding to the precursor amino acid sequence.

The invention further provides an improved cellulase comprising an amino acid sequence which has been modified from a precursor amino acid sequence having the sequence of *H. grisea* EGIII cellulase shown in Fig. 11 by substitution of one or more amino acid residues, said substitution being selected from: C210V and C170G, said amino acid numbering being as in Figure 11; wherein the improved cellulase has increased thermostability compared to a cellulase corresponding to the precursor amino acid sequence.

The invention further provides a DNA sequence encoding a cellulase of the invention.

The invention further provides an expression vector comprising a DNA sequence of the invention.

The invention further provides a host cell comprising a DNA sequence of the invention or an expression vector of the invention.

The invention further provides a method of producing a cellulase of the invention, comprising:

(a) modifying a DNA sequence encoding the precursor amino acid sequence to produce a modified DNA sequence consisting of the sequence as defined in accordance with the DNA sequence of the invention;

(b) expressing the modified DNA sequence in a host cell; and

(c) purifying the expressed cellulase protein.

The invention further provides use of a cellulase of the invention in biomass reduction, cleaning products or textile treatment compositions.

## BRIEF DESCRIPTION OF THE DRAWINGS

Figure 1 illustrates mutagenic oligonucleotides useful during directed mutagenesis of Asn188 from *Bacillus licheniformis* α-amylase. In this and following figures illustrating oligonucleotide constructs, bold letters indicate base changes introduced by the oligonucleotide and underlining indicates restriction endonuclease sites introduced by the oligonucleotide.

Figure 2 illustrates PCR primers used for PCR processing of mutagenic oligonucleotide templates.

Figure 3 illustrates the DNA sequence of the gene for α-amylase from *Bacillus licheniformis* (NCIB 8061) (SEQ ID NO:33) and deduced amino acid sequence of the translation product (SEQ ID NO:41) as described by Gray et al., J. Bacteriology, vol. 166, pp. 635-643 (1986).

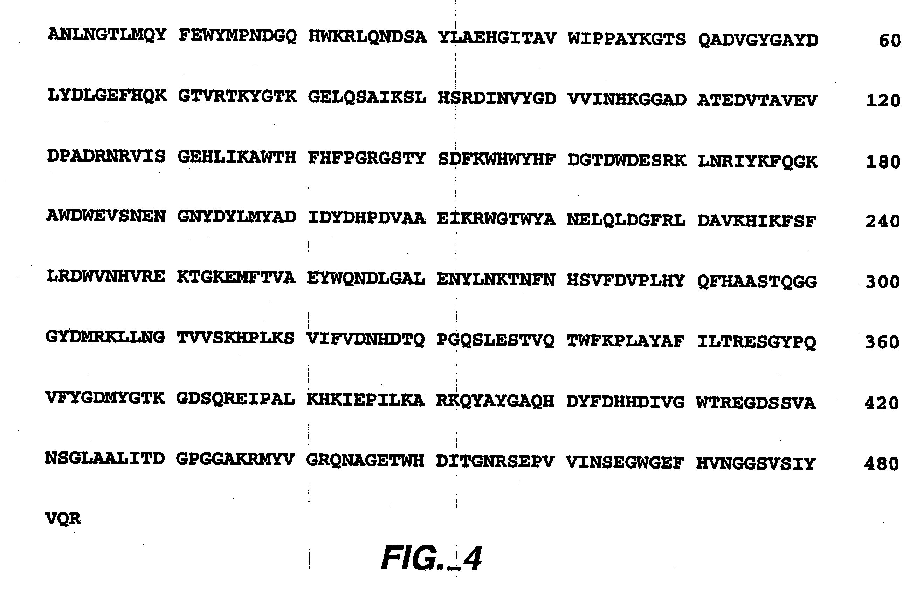

Figure 4 illustrates the amino acid sequence (SEQ ID NO:34) of the mature α-amylase enzyme from *Bacillus licheniformis.*

Figure 5 illustrates an alignment of the primary structures of three *Bacillus* α-amylases. The *Bacillus licheniformis* α-amylase (Am-Lich) (SEQ ID NO:35) is described by Gray et al., J. Bacteriology, vol. 166, pp. 635-643 (1986); the *Bacillus amyloliquefaciens* α-amylase (Am-Amylo) (SEQ ID NO:36) is described by Takkinen et al., J. Biol. Chem., vol. 258, pp. 1007-1013 (1983); and the *Bacillus stearothermophilus* α-amylase (Am-Stearo) (SEQ ID NO:37) is described by Ihara et al., J. Biochem., vol. 98, pp. 95-103 (1985).

Figure 6 illustrates plasmid pHP13 wherein $Cm^R$ refers to chloramphenicol resistance, $Em^R$ refers to erythromycin resistance and Rep pTA1060 refers to the origin of replication from plasmid pTA1060.

Figure 7 illustrates the pBLapr plasmid wherein BL AA refers to *Bacillus licheniformis* α-amylase gene; *aprE* refers to the promoter and signal peptide encoding region of the *aprE* gene; AmpR refers to the ampicillin resistant gene from pBR322; and CAT refers to the chloramphenicol resistance gene from pC194.

Figure 8 illustrates the pHP.BL plasmid carrying the gene for *Bacillus licheniformis* α-amylase.

Figure 9 illustrates a schematic of the PCR method used to produce the mutant oligonucleotides corresponding to α-amylase derived from *Bacillus licheniformis.*

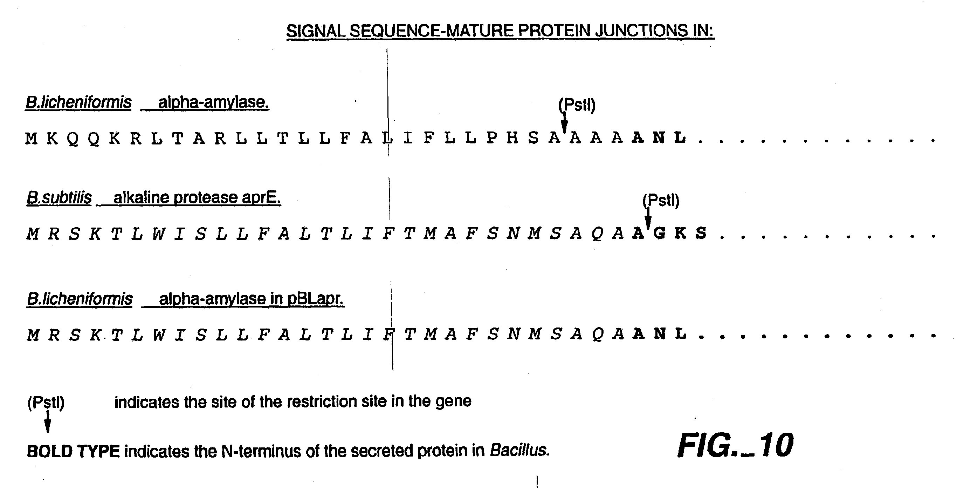

Figure 10 illustrates the signal sequence-mature protein junctions in α-amylase derived from *Bacillus licheniformis* (SEQ ID NO:38), *Bacillus subtilis aprE* (SEQ ID NO: 39) and *Bacillus licheniformis* in pBLapr (SEQ ID NO:40).

Figure 11 illustrates a sequence alignment of 8 homologous endoglucanase enzymes corresponding to certain cellulases obtained from *Trichoerma reesei (EGIII)(SEQ.* ID NO: 41), *Hypocrea schweinitzii* (SEQ. ID. NO: 42), *Aspergillus aculeatus* (SEQ. ID. NO: *43), Aspergillus kawachii* (SEQ. ID. NO: 44), *Humicola grisea* (SEQ ID NO:45), *Gliocladium roseum (1)* (SEQ. ID. NO:46), *Gliocladium roesum (2)* (SEQ. ID. NO: 47), *Gliocladium roseum (3)* (SEQ. ID. NO:48). Numbering provided is based on the numbering in EGIII.

## DETAILED DESCRIPTION

"Expression vector" means a DNA construct comprising a DNA sequence which is operably linked to a suitable control sequence capable of effecting the expression of said DNA in a suitable host. Such control sequences may include a promoter to effect transcription, an optional operator sequence to control such transcription, a sequence encoding suitable mRNA ribosome-binding sites, and sequences which control termination of transcription and translation. Where expression is desired in a *Bacillus* host, a preferred promoter is the *Bacillus subtilis aprE* promoter. The vector may be a plasmid, a phage particle, or simply a potential genomic insert. Once transformed into a suitable host, the vector may replicate and function independently of the host genome, or may, in some instances, integrate into the genome itself. While plasmid and vector are sometimes used interchangeably as the plasmid is the most commonly used form of vector at present, the invention is intended to include such other forms of expression vectors which serve equivalent functions and which are, or become, known in the art.

"Host strain" or "host cell" means a suitable host for an expression vector comprising DNA encoding a protein according to the present invention. Host cells useful in the present invention are generally procaryotic or eucaryotic non-human hosts, including any transformable microorganism in which the expression of a given protein according to the present invention can be achieved. One of skill in the art will be familiar with the appropriate expression and secretion machinery, including the appropriate host cell, for use with a specific protein. For example, host strains of the same species or genus from which the particular protein is derived are suitable, for example with α-amylase derived from *Bacillus,* a suitable host cell would be a *Bacillus* strain. In this case, an α-amylase negative *Bacillus* strain (genes deleted) and/or an α-amylase and protease deleted *Bacillus* strain (Δ*amyE,* Δ*apr*, Δ*npr*) is preferably used. Host cells are transformed or transfected with vectors constructed using recombinant DNA techniques. Such transformed host cells are capable of either replicating vectors encoding the protein and its variants (mutants) or expressing the desired protein.

"Recombinant protein" means a protein in which the DNA sequence encoding the naturally occurring or precursor protein is modified to produce a mutant DNA sequence which encodes the substitution, insertion or deletion of one or more amino acids in the protein sequence compared to a naturally occurring or precursor protein. As used herein, the term precursor protein (or enzyme) refers not to an immediate precursor in terms of a chemical reaction, but instead to a parent protein from which the modification is modeled. Accordingly, while it is the precursor which defines the modification, the actual alteration or modification will have its basis in an alteration within the DNA which encodes the precursor, which DNA is then transformed, expressed and the protein product secreted which incorporates the modification.

The improved protein according to the present invention comprises an amino acid sequence which is derived from the amino acid sequence of a precursor protein (also referred to herein as the "first protein"), the specific modifications in the precursor protein being as defined in the claims. The precursor protein may be a naturally occurring protein or a recombinant protein. The amino acid sequence of the improved protein is derived from the precursor protein's amino acid sequence by the substitution, deletion or insertion of one or more amino acids of the precursor amino acid sequence. Such modification is generally of the precursor DNA sequence which encodes the amino acid sequence of the precursor proteins rather than manipulation of the precursor protein *per se.* Suitable methods for such manipulation of the precursor DNA sequence include methods disclosed herein and in commonly owned U.S. Patent Nos. 4,760,025 and 5,185,258.

"Desired properties" means any property for which it is desired to improve a protein. Such desired properties may comprise functional properties such as thermal, pH, alkaline, oxidative or chemical stability, catalytic activity, substrate binding, receptor binding, allergenicity, antimicrobial activity or any other desirable feature associated with a protein.

"Primary amino acid sequence alignment" means an alignment of the primary sequence of at least two proteins in terms to produce a significant degree of sequence identity. Different alignment algorithms exist in the form of commercially available software which are suitable and are routine in use to the person of ordinary skill in the art. These programs provide for the input of different algorithm parameters which may effect the results obtained. However, the present invention does not require a preference for any specific sequence alignment algorithm as the choice is generally one of optimizing the parameters. In general, alignments are considered approximate, and thus using different methods of optimizing the alignment should advantageously provide slightly different pools of mutants to select from. Nonetheless, because an advantage of the present invention is to produce a focused pool of mutant proteins from which to select improved proteins, the present invention should be equally applicable regardless of the program or parameters used to determine the alignment.

A protein is modified as follows to improve its performance in terms of a desired property. The primary amino acid sequence of the protein to be modified, called the "first protein", is aligned with the primary amino acid sequence of a comparative "second homologous protein" which has less desirable performance in terms of the desired property. From the alignment, positions at which residues differ are determined and the residues present in the second homologous protein sequence at those positions are identified. At least one of the selected residues from the previous step is then incorporated into the amino acid sequence of the first protein. As surprisingly determined by the inventors hereof, the modified protein produced in this manner has a significant probability of having improved performance in terms of the desired property. Accordingly, a pool of mutant proteins produced in this manner having various combinations of identified residues substituted, deleted or added will, in all likelihood, comprise a pool of improved proteins which can then be selected among for the "winner".

In a variation, it is possible to produce alignments of more than two primary amino acid sequences. The first protein is aligned with more than one second homologous protein primary amino acid sequence, each second homologous protein primary amino acid sequence corresponding to a different protein which has less beneficial activity than that of the first protein. From this alignment residues are selected which exist in one or more of the second homologous proteins at positions in which there is a difference between the first protein primary amino acid sequence and at least two of the second homologous protein primary amino acid sequences. In one alternative which expands the pool of mutants available, each of the variations between the first protein a second homologous protein can be used as the basis for producing a mutant. In another preferred alternative which limits the number of mutants available to those most likely to successfully impart improved functional characteristics on the mutant proteins selected, only those differences in which each of the second homologous protein sequences comprise an identical residue are selected.

In one variation of the above method, the alignment is prepared with two or more homologous proteins which are not directly compared to the first protein. For example, two comparative homologous proteins, each of which are capable of being aligned with the first protein, are aligned to determine where residues differ. The resulting alignment is then analyzed to determine the residues which exist in the comparative protein or proteins having less desirable performance in terms of the desired property, and that residue is then substituted, deleted or added into the first protein at a corresponding position. It is not necessary that the comparative proteins be better or worse than the first protein, only that the selected residue for modification in the target protein is derived from the homologous comparative protein which has less desirable performance in terms of the desired property. In addition, it is possible to align more than two second homologous protein primary amino acid sequences and to select from among them based on the performance of the various second homologous proteins in terms of the desired property.

Applicants have surprisingly and unexpectedly found that a protein having an improved functional property may be obtained by preparing an alignment of two or more homologous or related proteins and referencing the residues which exist at various locations in the proteins having less desirable performance in terms of a desired property. For example, by comparing a first protein against a second homologous protein that has less desirable characteristics in terms of the functional property, identifying residues which differ, preparing a pool of mutants based on said differing mutations, and selecting the mutants which are improved in terms of the desired property in comparison with the first protein, advantageous improvements in desired properties may be obtained in a significant percentage of such modifications. Thus, one excellent use is to produce a relatively small and manageable pool of mutants (in comparison with the pool of random mutants) from which it is possible to select a protein having significantly improved functional properties when compared to the first protein. Moreover, this result is possible without having intimate structure-function information.

It is not necessary that the aligned proteins have comparable activity and function. In a preferred embodiment, however, the proteins have comparable activity, i.e., similar biological activity, function, catalytic activity or such other criteria as are commonly used to classify a specific protein. However, determining positions at which nature has determined a residue can exist in the context of a protein without significantly effecting its value to a host organism, it is not necessary that all features of the compared proteins be identical, merely that they have some homology. "Substantially homologous" means that the proteins have a significant level of conserved, i.e., identical, amino acids such that their sequences can be meaningfully aligned and major structural, functional or catalytic sites defined. Preferably, the first protein and the second homologous proteins have a sequence identity of at least 30%, preferably 50% sequence identity, more preferably 70% sequence identity and most preferably 85% sequence identity.

"α-Amylase" as used herein means an enzymatic activity which cleaves or hydrolyzes the α(1-4)glycosidic bond, e.g., that in starch, amylopectin or amylose polymers. α-Amylase includes naturally occurring α-amylases as well as recombinant α-amylases. Preferred α-amylases in the present invention are those derived from *Bacillus sp.,* particularly those from *Bacillus licheniformis, Bacillus amyloliquefaciens* or *Bacillus stearothermophilus,* as well as fungal α-amylases such as those derived from *Aspergillus* (i.e., A. *oryzae* and A. *niger*). The precursor α-amylase is produced by any source capable of producing α-amylase. Suitable sources of α-amylases are prokaryotic or eukaryotic organisms, including fungi, bacteria, plants or animals. Preferably, the precursor α-amylase is produced by a *Bacillus;* more preferably, by *Bacillus licheniformis, Bacillus amyloliquefaciens* or *Bacillus stearothermophilus;* most preferably, the precursor α-amylase is derived from *Bacillus licheniformis.*

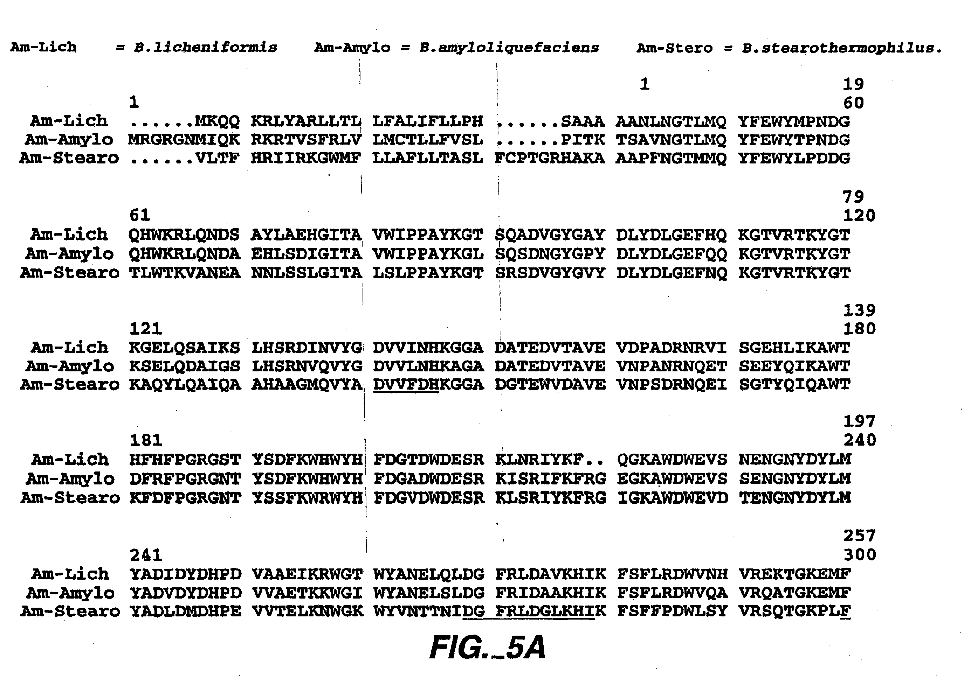

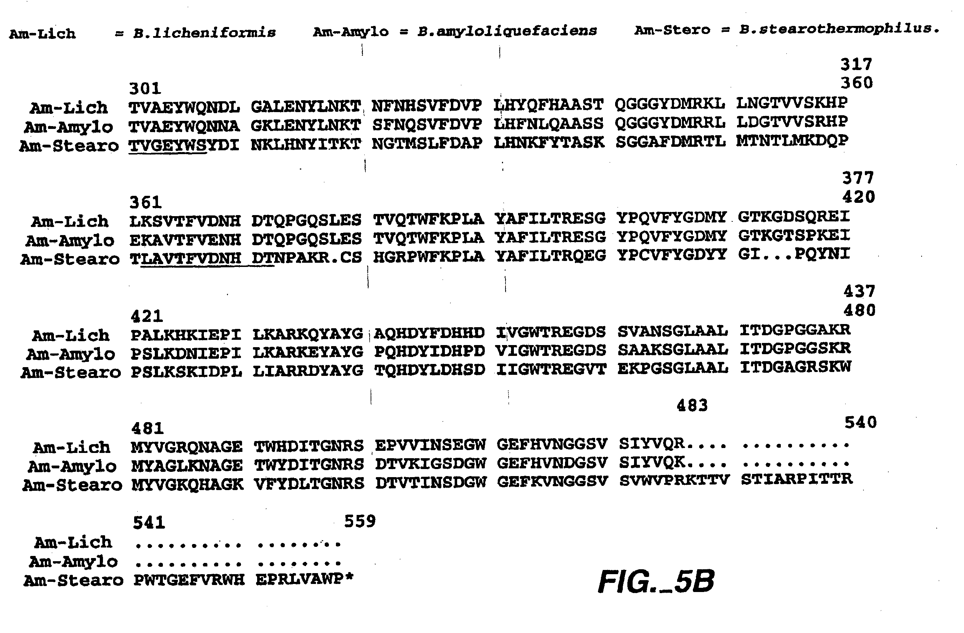

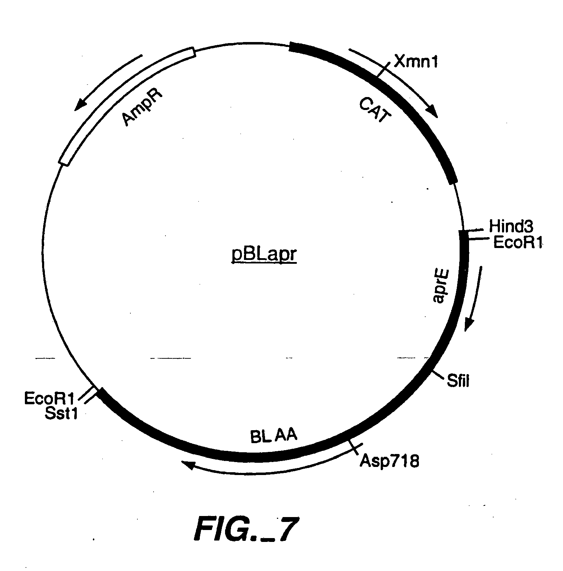

Homologies have been found between almost all endo-amylases sequenced to date, ranging from plants, mammals, and bacteria (Nakajima et al., Appl. Microbiol. Biotechnol., vol. 23, pp. 355-360 (1986); Rogers, Biochem. Biophys. Res. Commun., vol. 128, pp. 470-476 (1985); Janecek, Eur. J. Biochem., vol. 224, pp. 519-524 (1994)). There are four areas of particularly high homology in certain *Bacillus* amylases, as shown in Figure 5, wherein the underlined sections designate the areas of high homology. Sequence alignments have also been used to map the relationship between *Bacillus* endo-amylases (Feng et al., J. Molec. Evol., vol. 35, pp. 351-360 (1987)). The relative sequence homology between *Bacillus stearothermophilus* and *Bacillus licheniformis* amylase is about 66% and that between *Bacillus licheniformis* and *Bacillus amyloliquefaciens* amylases is about 81%, as determined by Holm et al., Protein Engineering, vol. 3, No. 3, pp. 181-191 (1990). In order to establish homology to primary structure, the amino acid sequence of a precursor α-amylase is directly compared to the *Bacillus licheniformis* α-amylase primary sequence and particularly to a set of residues known to be invariant to all α-amylases for which sequences are known (see e.g., Figure 7). It is possible also to determine equivalent residues by tertiary structure analysis of the crystal structures reported for porcine pancreatic α-amylase (Buisson et al., EMBO Journal, vol. 6, pp. 3909-3916 (1987); Qian et al., Biochemistry, vol. 33, pp. 6284-6294 (1994); Larson et al., J. Mol. Biol., vol. 235, pp. 1560-1584 (1994)); Taka-amylase A from *Aspergillus oryzae* (Matsuura et al., J. Biochem. (Tokyo), vol. 95, pp. 697-702 (1984)); and an acid α-amylase from *A. niger* (Boel et al.. Biochemistry, vol. 29, pp. 6244-6249 (1990)), with the former two structures being similar, and for barley α-amylase (Vallee et al., J. Mol. Biol., vol. 236, pp. 368-371(1994); Kadziola, J. Mol. Biol., vol. 239, pp. 104-121 (1994)). Although there have been some preliminary studies published (Suzuki et al, J. Biochem., vol. 108, pp. 379-381 (1990); Lee et al., Arch. Biochem. Biophys, vol. 291, pp. 255-257 (1991); Chang et al, J. Mol. Biol., vol. 229, pp. 235-238 (1993); Mizuno et al., J. Mol. Biol., vol. 234, pp. 1282-1283 (1993)), there is only a published structure for *Bacillus licheniformis* α-amylase (Machius et al., J. Mol. Biol. vol. 246, pp. 545-549 (1995)). However, several researchers have predicted common super-secondary structures between glucanases (MacGregor et al., Biochem. J., vol. 259, pp. 145-152 (1989)) and within α-amylases and other starch-metabolising enzymes (Jaspersen, J. Prot. Chem. vol. 12, pp. 791-805 (1993); MacGregor, Starke, vol. 45, pp. 232-237 (1993)); and sequence similarities between enzymes with similar super-secondary structures to α-amylases (Janecek, FEBS Letters, vol. 316, pp. 23-26 (1993); Janecek et al., J. Prot. Chem., vol. 12, pp. 509-514 (1993)). A structure for the *Bacillus stearothermophilus* enzyme has been modeled on that of Taka-amylase A (Holm et al., Protein Engineering, vol. 3, pp. 181-191 (1990)). The four highly conserved regions shown in Figure 7 contain many residues thought to be part of the active-site (Matsuura et al., J. Biochem. (Tokyo), vol. 95, pp. 697-702 (1984); Buisson et al., EMBO Journal, vol. 6, pp. 3909-3916 (1987); Vihinen et al., J. Biochem., vol. 107, pp. 267-272 (1990)) including His +105; Arg +229; Asp +231; His +235; Glu +261 and Asp +328 under the *Bacillus licheniformis* numbering system. The crystal structure of a bacterial amylase hybrid enzyme has been published in PCT Publication No. WO 96/23874.

As described above, the α-amylases from *Bacillus licheniformis, Bacillus stearothermophilus, Bacillus amyloliquefaciens* and *Bacillus subtilis* all bear a significant degree of homology. However, it is known that under the conditions of industrial starch liquefaction, e.g., high temperature (excess of 90°C), low pH (pH 4-6) and low calcium, α-amylase derived from *Bacillus licheniformis* provides the most acceptable performance. Nonetheless, even *Bacillus licheniformis* α-amylase is susceptible to undesirable instability under liquefaction conditions making a more stable alternative desirable. Accordingly, much work has been performed on the *Bacillus licheniformis* derived molecule for the purpose of introducing into this enzyme properties which are desirable in connection with its use in liquefaction processes. By aligning the sequence of α-amylase from *Bacillus licheniformis* against that of α-amylase from either *Bacillus stearothermophilus* or *Bacillus amyloliquefaciens* it is possible to identify residues which differ between the homologs. The positions at which residues differ in α-amylase from *B. stearothermophilus* or *B. amyloliquefaciens* compared to α-amylase from *B. licheniformis* are selected for substitution in α-amylase from *B. licheniformis.* Preferably, the substituted residue is actually an identical residue as that which exists in α-amylase from either *B. stearothermophilus* or *B. amyloliquefaciens.* More preferably, the substituted residue corresponds to a position in which the same residue exists in both α-amylase from *B. stearothermophilus* and *B. amyloliquefaciens.*

Residues specifically identified herein for replacement in *Bacillus licheniformis* are those which differ from residues in a corresponding position in *Bacillus amyloliquefaciens* and/or *Bacillus stearothermophilus,* and particularly A33, A52, S85, N96, H133, S148, A209, A269, A379 and A435. Specific preferred replacements for these residues are selected from those present in both *Bacillus amyloliquefaciens* and *Bacillus stearothermophilus* and correspond to A33S, A52S, N96Q, H133Y, S148N, A209V, A269K, A379S and/or A435S all appear to contribute to a stability benefit. It has also been discovered that the A85D mutation, which is recruited only from *Bacillus amyloliquefaciens* also provides a stability benefit. The above residues may be altered in combination with other modifications which provide a performance benefit.

Enhanced thermostability will be useful in extending the shelf life of products which incorporate them and for applications at high temperatures. Enhanced oxidative stability or improved performance is particularly desirable in cleaning products, and for extending the shelf life of the enzyme in the presence of bleach, perborate, percarbonate or peracids used in such cleaning products. Cellulases having improved stability benefits are also disclosed herein and are particularly useful in, for example, biomass reduction, cleaning products and textile treatment compositions.

An additional embodiment of the present invention comprises DNA encoding a protein according to the present invention and expression vectors comprising such DNA. The DNA sequences may be expressed by operably linking them to an expression control sequence in an appropriate expression vector and employing that expression vector to transform an appropriate host according to well known techniques. A wide variety of host/expression vector combinations may be employed in expressing the DNA sequences of this invention. Useful expression vectors, for example, include segments of chromosomal, non-chromosomal and synthetic DNA sequences, such as the various known plasmids and phages useful for this purpose. In addition, any of a wide variety of expression control sequences are generally used in these vectors. For example, with α-amylase derived from *Bacillus,* Applicants have discovered that a preferred expression control sequence for *Bacillus* transformants is the *aprE* signal peptide derived from *Bacillus subtilis.*

A wide variety of host cells are also useful in expressing the DNA sequences of this invention. These hosts may include well known eukaryotic and prokaryotic hosts, such as strains of *E. coli, Pseudomonas, Bacillus, Streptomyces,* various fungi, yeast and animal cells. Preferably, the host expresses the protein of the present invention extracellularly to facilitate purification and downstream processing. Expression and purification of the improved protein of the invention may as defined in the claims be effected through art-recognized means for carrying out such processes.

The following is presented by way of example and is not to be construed as a limitation to the scope of the claims. Abbreviations used herein, particularly three letter or one letter notations for amino acids are described in Dale, J.W., Molecular Genetics of Bacteria, John Wiley & Sons, (1989) Appendix B.

## EXAMPLES

Comparative EXAMPLE 1

### Construction Of Plasmid pHP.BL

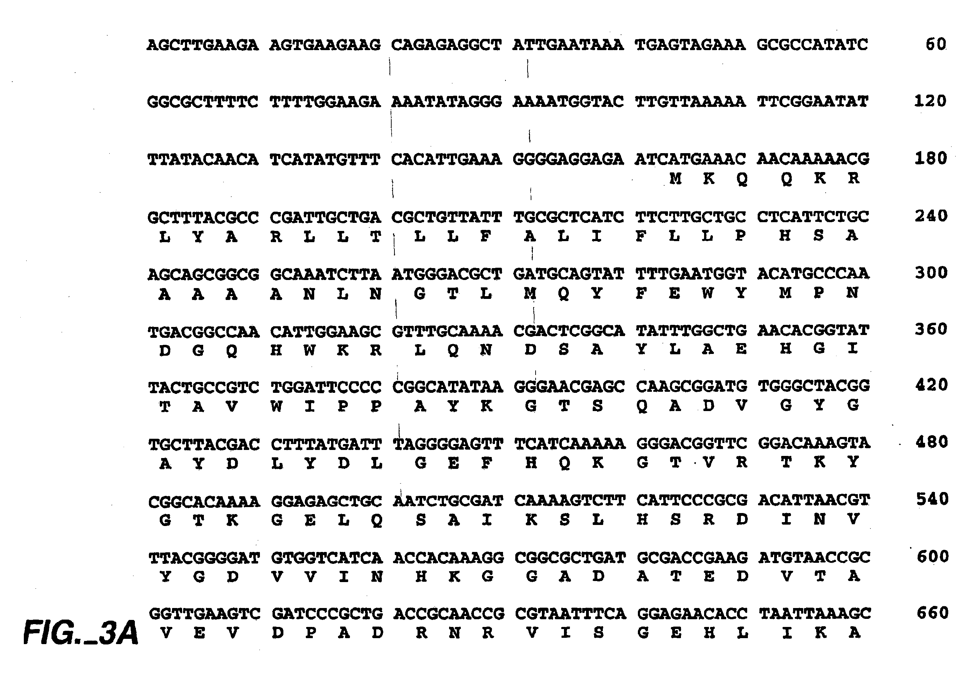

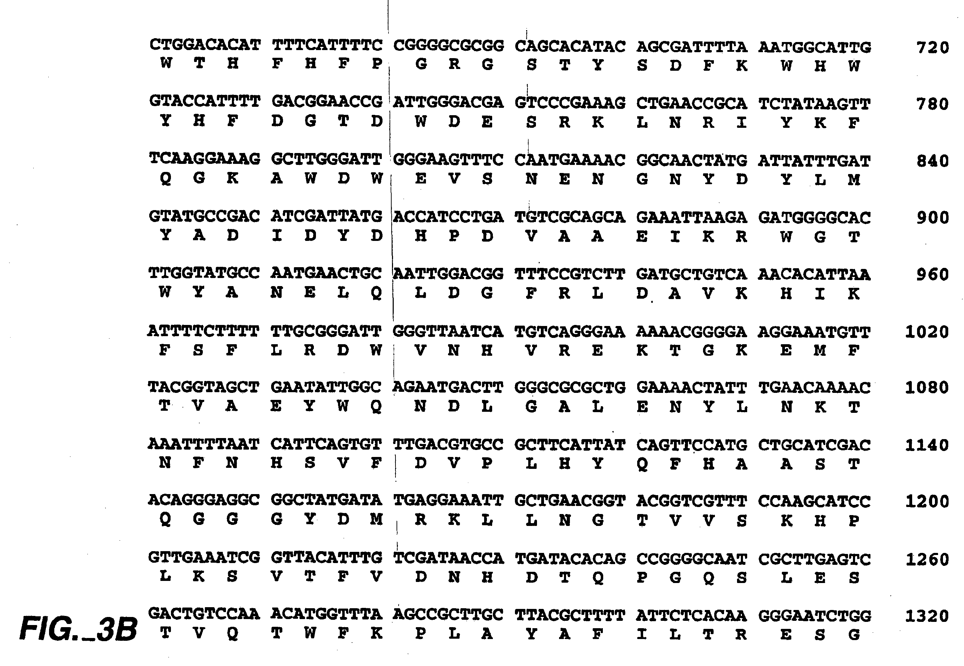

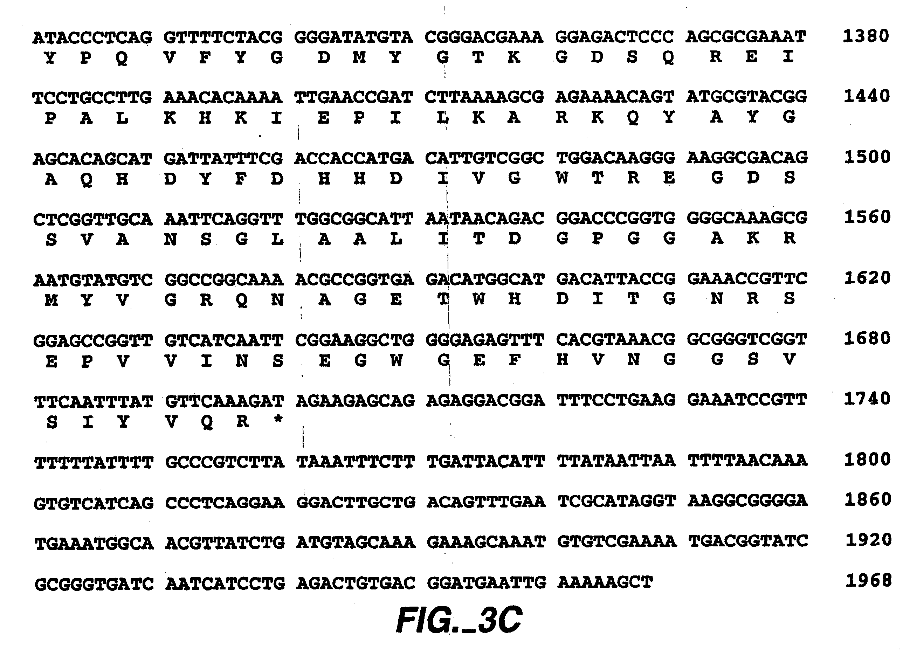

The α-amylase gene shown in Figure 3 was cloned from *Bacillus licheniformis* NCIB8061 (Gray et al., J. Bacteriology, vol. 166, pp. 635-643 (1986)). The 1.72kb PstI-SstI fragment, encoding the last three residues of the signal sequence, the entire mature protein and the terminator region, was subcloned into M13mp18. A synthetic terminator was added between the BclI and SstI sites using a synthetic oligonucleotide cassette of the form:

| | |
|---|---|
| BclI | SstI |
| 5'-GATCAAAACATAAAAAACCGGCCTTGGCCCCGCCGGTTTTTTATTATTTTTGAGCT-3' | (SEQ ID NO:1) |
| 3' TTTTGTATTTTTTGGCCGGAACCGGGGCGGCCAAAAAATAATAAAAAC 5' | (SEQ ID NO:2) |

designed to contain the *Bacillus amyloliquefaciens* subtilisin transcriptional terminator (Wells et al., Nucleic Acid Research, vol. 11, pp. 7911-7925 (1983)).

The pBLapr plasmid was constructed carrying the gene for the *Bacillus licheniformis* α-amylase. As illustrated in Figure 7, pBLapr comprises a 6.1 kb plasmid including the ampicillin resistance gene from pBR322 and the chloramphenicol resistance gene from pC194, the aprE promoter and the gene encoding for the *Bacillus licheniformis* α-amylase ("BL AA"). The aprE promoter was constructed from a 660bp HindIII-PstI fragment encoding for the promoter and signal sequence of the *Bacillus subtilis* alkaline protease. The PstI site was removed, and an SfiI site added close to the *aprE*/BL AA junction. The BL AA gene comprises the 1720 bp PstI-SstI fragment described above. In the work described herein, pBLapr was constructed with an SfiI site adjacent to the 5' end of the start of the coding sequence for the mature amylase gene. Specifically, the 5' end of the pBLapr construction was subcloned on an EcoRI-SstII fragment from pBLapr into M13BM20 (Boehringer Mannheim) to obtain a coding-strand template for the mutagenic oligonucleotide below:

5'- CCC ATT AAG ATT <u>GGC CGC CTG GGC C</u>GA CAT GTT GCT GG - 3' (SEQ ID NO:3)

This primer introduced an SfiI site (indicated by underlining) which allowed correct forms to be screened for by the presence of this unique restriction site. Subcloning the EcoRI-SstII fragment back into the pBLapr vector gave a version of the plasmid containing an SfiI site.

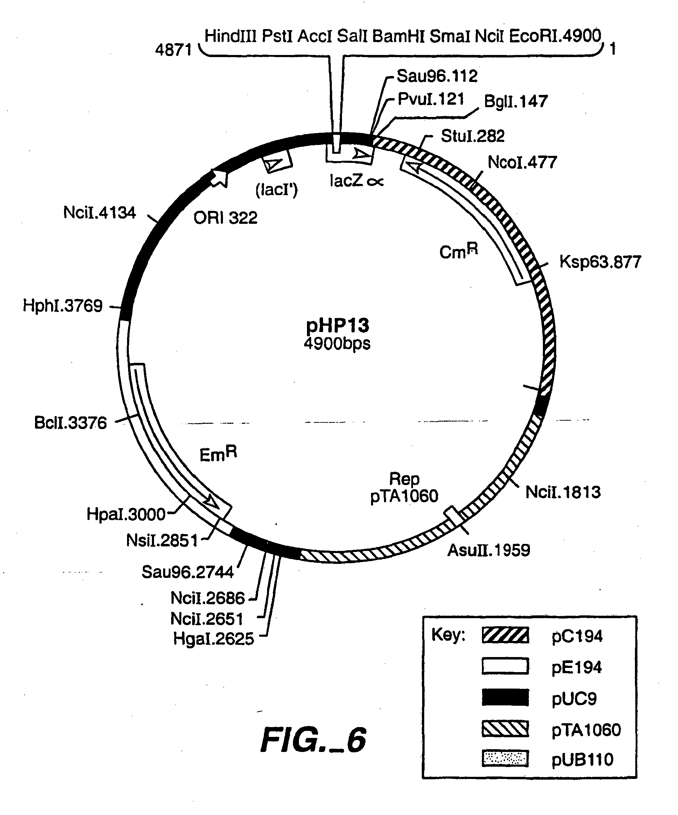

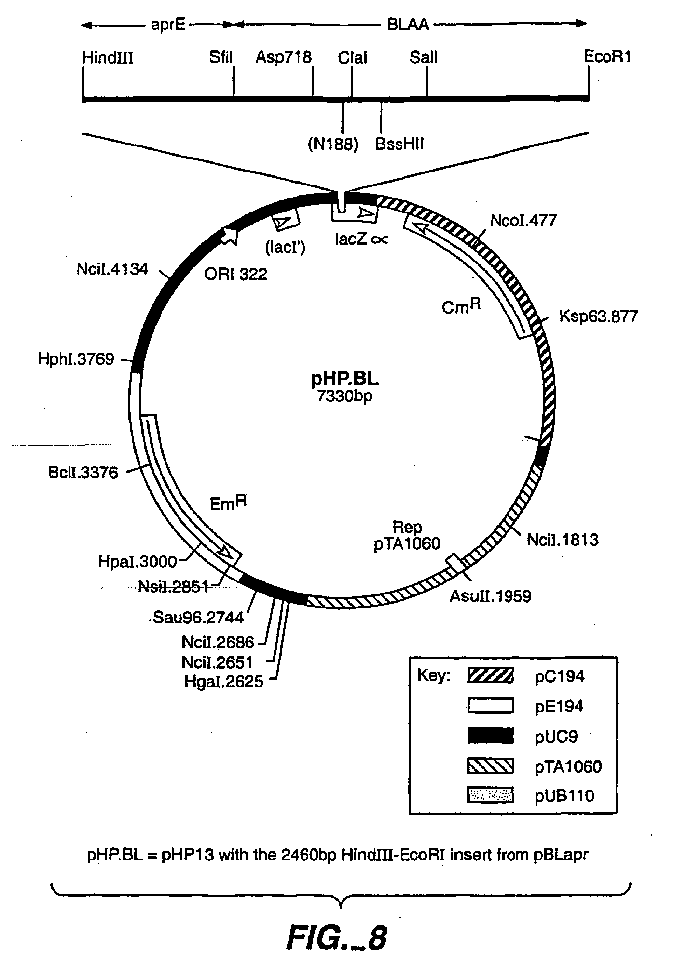

Plasmid pHP13 (Haima et al., Mol. Gen. Genet., vol. 209, pp. 335-342 (1987)) (Figure 6) was digested with restriction enzymes EcoRI and HindIII and the resulting vector purified on a polyacrymide gel and then eluted. Plasmid pBLapr was digested with HindIII, Asp718 and in a separate incubation with Asp718, EcoRI and gel purified. Two bands, HindIII-Asp718 (1203 bp) and Asp718-EcoRI (1253 bp)were gel purified, eluted from the gel and ligated into the vector by a 3-way ligation, to give plasmid pHP.BL, the plasmid used in expression of the α-amylase (Figure 8).

Comparative EXAMPLE 2

### Construction Of Plasmid Encoding α-Amylase Comprising Substitutions For Asparagine 188

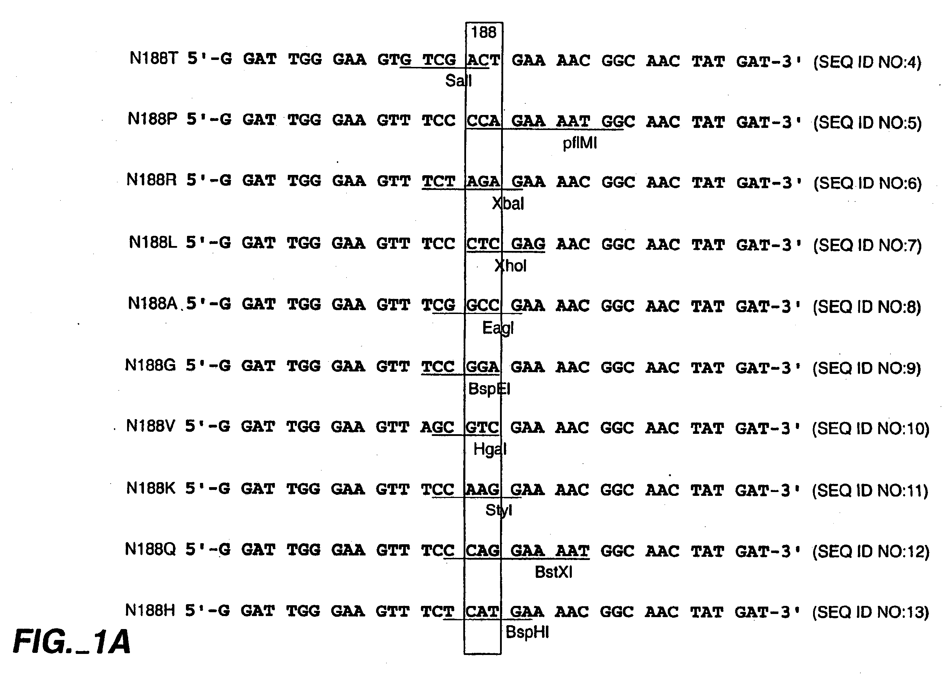

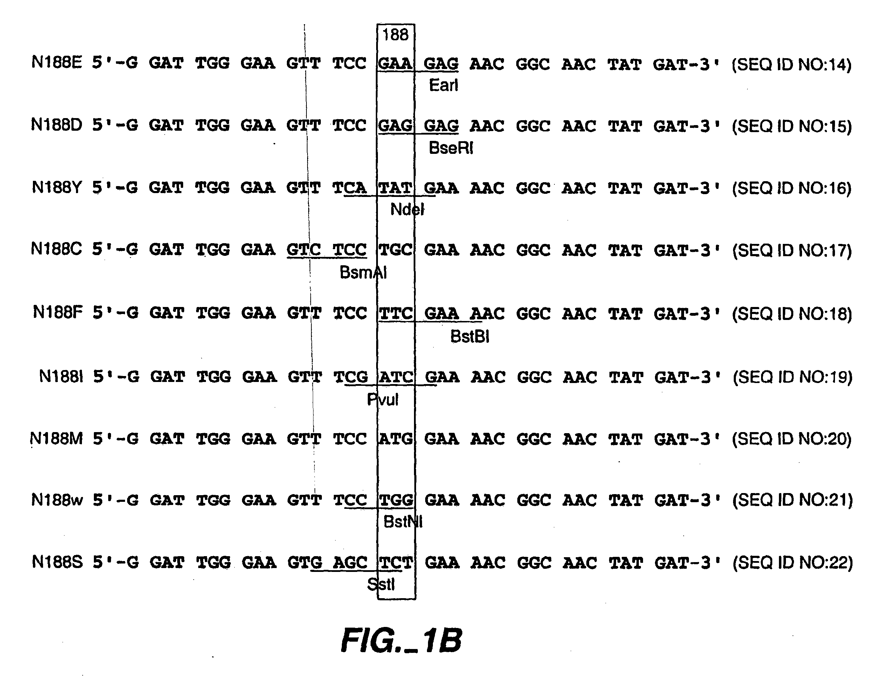

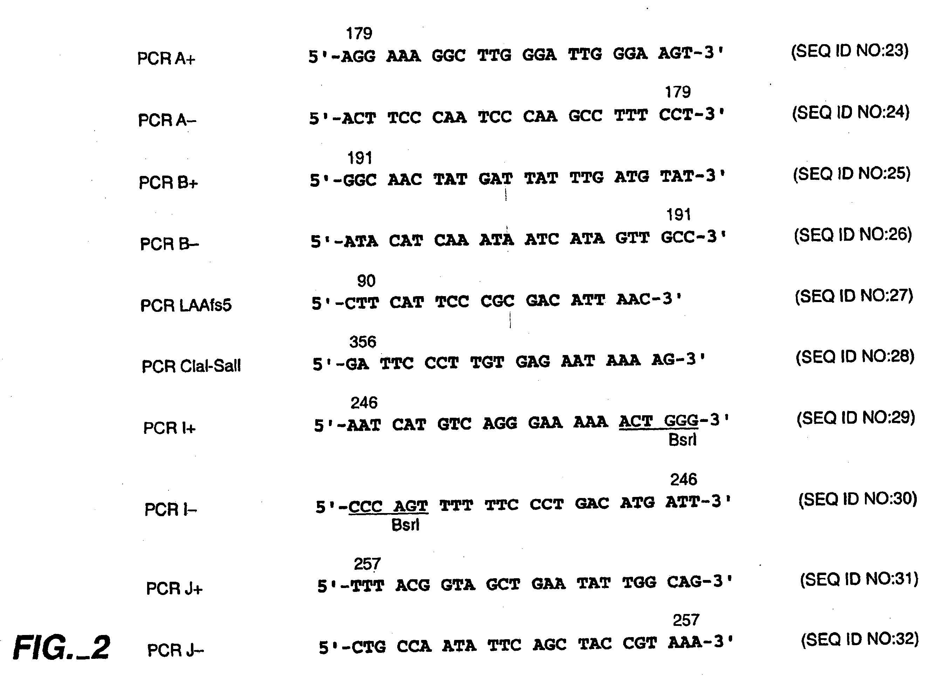

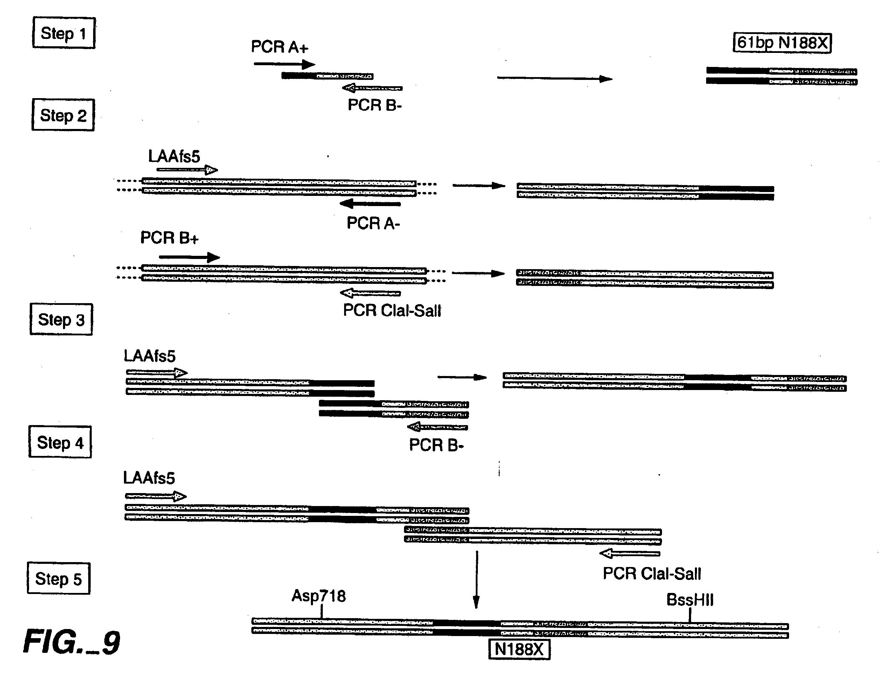

A series of mutagenic primers encoding for substitutions of Asn188 ("N188") with each of the naturally occurring amino acids were synthesized and are shown in Figure 1 (SEQ ID NOS:4-22). The α-amylase gene mutations encoding for these changes were made by PCR, according to the procedure summarized in Figure 9, using the PCR primers shown in Figure 2 (SEQ ID NOS:23-32).

Step (1): The mutagenic primers were used as templates for the PCR primers PCR A+ and PCR B- resulting in a lengthened (61 bp) double stranded DNA. Each contained a different amino acid replacement at position 188, and all except N188M contained a different restriction site. Initially the PCR primers were annealed at 35° C for five minutes followed by a one minute DNA extension with taq polymerase at 75° C. The double stranded DNA was then melted at 95° C for one minute, followed by the annealing and extension steps. Melting, annealing and extension continued for a total of 30 cycles.

Step (2): DNA upstream and downstream of position 188 were made in separate PCR reactions. The template was pBLapr, and the PCR primers were LAAfs5 (SEQ ID NO:27) and PCR A- (SEQ ID NO:24) for upstream; and PCR B+ (SEQ ID NO:25) and PCR Cla-SalI (SEQ ID NO:28) for downstream DNA. The DNA was melted at 95° C for one minute, annealed at 45° C for three minutes and elongated at 68° C for 3 minutes. The upstream portion is 290 bp and downstream is 498 bp. This procedure was repeated for 18 cycles using pfu polymerase. The same PCR procedure was used in steps (3) and (4).

Step (3): The upstream portion of DNA described in step (2) was attached to the double stranded mutagenic primers described in step (1). Primers LAAfs5 (SEQ ID NO:27) and PCR B- (SEQ ID NO:26) were used. As the result of primer design there is a 24 bp overlap between these templates allowing for the attachment of the two pieces of DNA.

Step (4): The downstream portions of DNA described in Step (2) and the product of Step (3) were attached to give the final product. A 24 bp overlap between the two PCR products allows for the attachment. Primers used were LAAfs5 (SEQ ID NO:27) and PCR ClaI-SalI (SEQ ID NO:28).

Step (5): Unique restriction sites, Asp718 and BssHII, are located upstream and downstream, respectively, of the 188 site. The final PCR product is digested with Asp718 and BssHII, the 333 bp fragment isolated by polyacrylamide gel electrophoresis and subcloned into the pHP.BL vector to obtain pHP.N188X.

Mutations were confirmed by dideoxy sequencing (Sanger et al., Proc. Natl. Acad. Sci. U.S.A., vol. 74, pp. 5463-5467 (1977)).

With reference to the DNA sequence and numbering system used in Figure 3, the codon encoding for the +188 amino acid position is at base pairs 812-814. PCR primers A+ and A- correspond to base pairs 784-807. PCR primers B+ and B- correspond to base pairs 821-844. The 5' end of PCR primer LAAfs5 corresponds to base pair 518. The 5' end of PCR primer PCR ClaI-SalI corresponds to base pair 1317. The Asp718 site corresponds to base pair 724. The BssHII site corresponds to base pair 1053.

Comparative EXAMPLE 3

Construction Of Plasmid Encoding Mutations At M15 And N188

A pBLapr plasmid having threonine substituted for methionine at amino acid 15 was constructed according to U.S. Patent Application Serial No. 08/194,664 (PCT Publication No. WO 94/18314). This plasmid (pBLaprM15T) was digested with SfiI and Asp718, and the 477 base pair fragment subcloned into pHP.BL to create pHP.M15T. In a manner analogous to that described above, Example 1, pHP.M15T was digested with Asp718 and BssHII, gel purified and eluted from the gel. The 333 base pair fragment comprising Asp718 to BssHII and the fragment from pHP.N188S were then subcloned into pHP.M15T to give plasmid pHP.M15T/N188S. In an analogous manner, starting with plasmids pBL aprM15L and pHP.NI88Y, the plasmid pHP.M15L/N188Y was constructed.

Comparative EXAMPLE 4

Transformation Of Plasmids Into *Bacillus subtilis,* Expression And Purification of Mutant $\alpha$-Amylase

$\alpha$-Amylase was expressed in *Bacillus subtilis* after transformation with the plasmids described in Examples 1-3. pHP13 is a plasmid able to replicate in *E. coli* and in *Bacillus subtilis.* Plasmids containing different variants were constructed using *E. coli* strain MM294, the plasmids isolated and then transformed into *Bacillus subtilis* as described in Anagnostopoulos et al., J. Bacter., vol. 81, pp. 741-746 (1961). The *Bacillus* strain had been deleted for two proteases ($\Delta$apr, $\Delta$npr) (see e.g., Ferrari et al., U.S. Patent No. 5,264,366) and for amylase ($\Delta$*amyE*) (see e.g., Stahl et al., J. Bacter., vol. 158, pp. 411-418 (1984)). The *bacillus* strain expressing M15L/N188Y was found to form larger zones of clearing than the strain expressing M15L on agar plates containing 1% insoluble starch indicating increased amylolytic activity. After transformation, the sacU(Hy) mutation (Henner et al., J. Bacter., vol., 170, pp. 296-300 (1988)) was introduced by PBS-1 mediated transduction (Hoch, J. Bact., vol. 154, pp. 1513-1515 (1983)).

Secreted amylases were routinely recovered from *Bacillus subtilis* cultures as follows: The culture supernatant was adjusted to 20% saturated ammonium sulfate and stirred for one hr. at 4°C. After centrifugation, the resultant supernatant was adjusted to 70% saturated ammonium sulfate and stirred for one hr. at 4°C. After centrifugation of the supernatant, the resultant pellet was re-dissolved in 50mM sodium acetate, pH 6.0, 5mM calcium chloride, and sterile filtered.

Comparative EXAMPLE 5

Assay For Determining α-Amylase Activity

Soluble Substrate Assay: A rate assay was developed based on an end-point assay kit supplied by Megazyme (Aust.) Pty. Ltd. A vial of substrate (p-nitrophenyl maltoheptaoside, BPNPG7) was dissolved in 10ml of sterile water followed by a 1:4 dilution in assay buffer (50mM maleate buffer, pH 6.7, 5mM calcium chloride, 0.002% Tween20). Assays were performed by adding 10μl of amylase to 790μl of the substrate in a cuvette at 25°C. Rates of hydrolysis were measured as the rate of change of absorbance at 410nm, after a delay of 75 seconds. The assay was linear up to rates of 0.2 absorption units/min.

α-Amylase protein concentration was measured using the standard Bio-Rad Assay (Bio-Rad Laboratories) based on the method of Bradford, Anal. Biochem., vol. 72, p. 248 (1976) using bovine serum albumin standards.

Starch Hydrolysis Assay: α-Amylase activity on starch was determined through an assay which depends on the ability of starch to form a blue colored complex with iodine and the disappearance of this color when starch is hydrolyzed to shorter dextrin molecules. The α-amylase activity was defined in terms of the digestion time required to produce a color change denoting a definite state of dextrination of the starch.

Reagents used were as follows:

*Phosphate buffer*- Potassium dihydrogen phosphate (340 g) and sodium hydroxide (25.3 g) were dissolved in water and diluted to - two liters. The buffer was cooled to room temperature and the pH was adjusted to 6.2±0.1. The buffer was diluted to two liters in a volumetric flask.

*Starch subsfrate* - Ten grams (dry substance) of soluble lintner starch were suspended in 50 ml of water and washed into ~300 ml of boiling water. The suspension was again brought to boiling and was boiled for five minutes with constant stirring. The starch solution was cooled with constant stirring to room temperature and 125 ml of phosphate buffer was added. The solution was diluted to 500 ml with water. The starch substrate was made fresh daily.

*Stock iodine solution* - Iodine crystals (5.5 g) and potassium iodide (11.0 g) were dissolved in water and were volumetrically diluted to 250 ml. The solution was kept from light.

*Dilute iodine solution* - Potassium iodide (20 g) and two ml of stock iodine solution were dissolved in water and diluted volumetrically to 500 ml. The solution was made fresh daily.

*Enzyme diluting solution* - Calcium chloride (11.1 g) was dissolved in four liters of water. Water used for all reagents was either distilled or deionized.

An α-amylase sample was diluted to between 10-15 LU/ml (as defined below) with enzyme diluting solution. For many commercial α-amylase preparations a suitable dilution was found to be 2000 fold. Five milliliter aliquots of dilute iodine solution were dispensed into 13 x 100 mm test tubes and 10 ml of starch substrate was placed in a 23 x 200 mm test tube. All tubes were placed in the 30°C water bath. A Hellige comparator equipped with a special α-amylase color disc (catalog number 620-s5) was used to make readings. Five milliliters of diluted enzyme (also at 30°C) were mixed with the starch substrate and timing was begun. At appropriate time intervals, for example one minute intervals early in the reaction and 15 second intervals later in the reaction, one ml aliquots of the enzyme-substrate mixture were transferred to a tube containing the dilute iodine solution. The starch iodine solution was mixed and transferred to a 13 mm precision square tube and the color was compared with the standard α-amylase color disc in the Hellige comparator. When the time of the end point was approached, samples were taken at 0.25 minute intervals.

The time required for the colors of the samples and the color disc to match were recorded and the activity (in liquefons per gram or ml) was calculated according to the formula:

$$\text{LU/ml or LU/g} =$$

Where:

LU =liquefon unit
V =volume of enzyme (5 ml or grams)
t =dextrinization time (minutes)
D =dilution factor:dilution volume divided by ml or g of enzyme diluted.

Comparative EXAMPLE 6

Preparation and Testing of Additional Mutant Alpha-Amylases for Thermal Stability

*Bacillus stearothermophilus* and *Bacillus amyloliquefaciens* produce an α-amylase which has poorer stability than the α-amylase produced by *Bacillus licheniformis.* From an alignment of these amylases, residues which differ in *B. stearothermophilus* or *B. amyloliquefaciens* compared to *B. licheniformis* were identified. From this analysis, mutant α-amylases based on the *B. licheniformis* sequence were prepared having substitutions at one or more of five positions for which corresponding residues in both *Bacillus stearothermophilus* and *Bacillus amyloliquefaciens* were identical but differed from *B. licheniformis:* A33S, A52S, N96Q S148N, A379S in combination with M15T/H133Y/N1885/A209V and compared with a mutant comprising only the M15T/H133Y/N1885/A209V substitutions. Additionally, the mutation S85D was incorporated which represents a recruitment only from the *Bacillus amyloliquefaciens* α-amylase. The mutations were prepared according to the procedures provided in Examples 1-4 except that appropriate PCR primers were provided to effect the desired mutations.

Thermal inactivation rates for the various mutants were measured according to the following procedure. Amylase stock solutions were dialysed extensively into 20 mM ammonium acetate, 4 mM $CaCl_2$ pH 6.5. For measurement of stability, this stock was diluted >50fold into a solution designed to induce rapid inactivation of wild type amylase: 50mM ammonium acetate, 5mM $CaCl_2$, 0.02% Tween 20 and a pH of 4.9, or 4.8 to a final concentration of between 30 and 50 μg/ml. Six 100μl aliquots were put into Eppendorf tubes and placed into a water bath at either 82°C or 83°C. The Eppendorf tubes were removed at regular, measured intervals of between 30 seconds and 5 minutes and placed on ice to stop the inactivation. The residual activity was assayed using a soluble substrate as described in Example 5. The natural log of the activity was plotted against time of incubation, and the rate constant for inactivation obtained from the slope of the straight line. The half-life was calculated as *ln*(2) divided by the rate constant. Results for various mutants are provided in Tables 1-4.

**TABLE 1**

| pH 4.9, 83°C | | |
|---|---|---|
| Variant | Inactivation rate constant ($Min^{-1}$) | $t_{1/2}$ (min.) |
| M15T/H133Y/N188S/A209V | 0.171 | 4.05 |
| M15T/D28N/A33S/H133Y/N 188S/A209V | 0.137 | 5.06 |
| M15T/A52S/H133Y/N188S/A209V/L230F | 0.144 | 4.81 |
| M15T/N96Q/H133Y/N188S/A209V/1479T | 0.162 | 4.27 |
| M15T/H133Y/S148N/N188S/A209V/G433D | 0.121 | 5.73 |
| M15T/H133Y/N188S/A209V/A379S | 0.145 | 4.78 |

**TABLE 2**

| pH 4.85, 83°C | | |
|---|---|---|
| Variant | Inactivation rate constant ($Min^{-1}$) | $t_{1l2}$ (min.) |
| M15T/H133Y/N188S/A209V | 0.252 | 2.75 |
| M15T/H133Y/N188S/A209V | 0.235 | 2.95 |
| M15T/H133Y/S148N/N188S/A209V/A379S | 0.114 | 6.05 |

**TABLE 3**

| pH4.85, 82°C | | |
|---|---|---|
| Variant | Inactivation rate constant ($Min^{-1}$) | $t_{1/2}$ (min.) |
| M15T/H133Y/N188S/A209V | 0.203 | 3.41 |
| M15T/H133Y/S148N/N188S/A209V/A379S | 0.106 | 6.54 |

(continued)

| pH4.85, 82°C | | |
|---|---|---|
| Variant | Inactivation rate constant ($Min^{-1}$) | $t_{1/2}$ (min.) |
| M15T/H133Y/N188S/A209V/A210S/T322A/A379S | 0.141 | 4.89 |
| M15T/H133Y/S148N/N188S/A209V | 0.122 | 5.65 |

TABLE 4

| pH4.80, 82.2°C | | |
|---|---|---|
| Variant | Inactivation rate constant ($Min^{-1}$) | $t_{1/2}$ (min.) |
| Wild Type | >2.0 | <0.35 |
| M15T/N188S | >1.9 | <0.36 |
| M15T/H133Y/N188S/A209V | 0.267 | 2.59 |
| M15T/S85D/H133Y/N188S/A209V | 0.236 | 2.93 |

## EXAMPLE 7

Recruitment of Residues From Less Stable Cellulases to More Stable Cellulases for Improvement of Stability of the More Stable Cellulase

Homologues of *Trichoderma reesei* EGIII cellulase from various species were obtained and tested for thermal stability. The cellulases were obtained from *(A) Hypocrea schweinitzii, (B)Aspergillus aculeatus, (C) Aspergillus kawachii, (D)Gliocladium roseum (1),(E) Gliocladium roseum (2), (F) Gilocladium roseum (3)and (G) Humicola grisea.* Of the above, cellulases (a) through (f) are less thermally stable than EGIII under the conditions of the assay. Cellulase (g) is more stable than EGIII. Accordingly, from the aligned sequences of these cellulases (as provided in Figure 11), it is possible to pick out the residues which exist in the less stable cellulase as compared to the more stable cellulase. To simplify reading of the table, positions marked for mutation are highlighted in bold. Mutants of EGIII were then prepared based on this information. In addition, mutant cellulase (g) was prepared based on recruitments from EGIII.

Equilibrium circular dichroism experiments were performed on an Aviv 62DS or 62ADS spectrophotometer, equipped with a 5 position thermoelectric cell holder supplied by Aviv. Buffer conditions were 50 mM bis-tris propane and 50 mM ammonium acetate adjusted to pH 8.0 with acetic acid. The final protein concentration for each experiment was in the range of 5-30 μM. Data was collected in a 0.1 cm path length cell.

Spectra were collected from 265-~210 nm. Thermal denaturations were performed at 217 nm from 30 to 90°C with data collected every two degrees. The equilibration time at each temperature was 0.1 minutes and data was collected for 4 seconds per sample.

The remainder of the pH 8.0 sample was divided into 5 x 400 uL aliquots. Two samples were adjusted to pH 5 and 7 with acetic acid and two others were adjusted to pH 9 and 10 with sodium hydroxide. Thermal denaturations of all samples were performed simultaneously as described above.

Table 5 illustrates 24 mutants which were prepared based on recruitments from less stable cellulases into EGIII and 3 mutants which were prepared based on recruitments from EGIII into a homolog isolated from *Humicola grisea* (note that the numbering in Table 5 for H.grisea is based on EGIII numbering in Figure 11). Of these, a significant number had improved stability over the wild type molecule (either EGIII or cellulase (g)) as shown in bold in Table 5.

TABLE 5

| | **Mutation** | **$\Delta T_m$** | **$T_m$ (°C)** | **Fit error** |
|---|---|---|---|---|
| PR | Product | | 54.10 | 0.09 |
| 0 | WT EG3 | | 54.60 | 0.18 |
| 1 | W7Y | -1.03 | 53.40 | 0.24 |
| 2 | **T11S/T16I** | 1.07 | 55.50 | 0.13 |

(continued)

| | Mutation | Δ $T_m$ | $T_m$ (°C) | Fit error |
|---|---|---|---|---|
| 3 | A35S | -4.03 | 50.40 | 0.14 |
| 4 | **S39N** | 0.47 | 54.90 | 0.17 |
| 5 | **G41A** | 2.47 | 56.90 | 0.11 |
| 6 | S63V | -0.83 | 53.60 | 0.11 |
| 7 | A66N | 0.07 | 54.50 | 0.10 |
| 8 | S77G | 0.07 | 54.50 | 0.09 |
| 9 | **N91D** | 0.47 | 54.90 | 0.17 |
| 10 | **S143T** | 0.47 | 54.90 | 0.12 |
| 11 | **T163S** | 0.27 | 54.70 | 0.07 |
| 12 | **N167S** | 0.17 | 54.60 | 0.10 |
| 13 | **A188G** | 0.47 | 54.90 | 0.17 |
| 14 | G31Q | -14.03 | 40.40 | 0.15 |
| 15 | Q162P | 0.07 | 54.50 | 0.19 |
| 16 | Y168F | -0.03 | 54.40 | 0.12 |
| 17 | **N174D** | 1.17 | 55.60 | 0.44 |
| 18 | V192L | -0.23 | 54.20 | 0.13 |
| 19 | N164D | -2.33 | 52.10 | 0.33 |
| 20 | **P201C** | 3.9/17.4 | 58.3/71.8 | 15/.23 |
| 21 | **V210C** | 0.47 | 70.60 | 70.80 |
| 22 | Q162P/T166P (17a* R2-2) | -1.43 | 53.00 | 0.29 |
| 23 | M79I | -7.13 | 47.30 | 0.27 |
| 24 | **T145E/Y147W** (25a4) | 0.77 | 55.20 | 0.05 |
| G0 | WT H. greisii | 0.00 | 68.5 | 0.11 |
| G1 | **C210V** | 1.50 | 70 | 0.2 |
| G2 | **C170G** | 2.10 | 70.6 | 0.23 |

## Claims

1. An improved cellulase comprising an amino acid sequence which has been modified from a precursor amino acid sequence having the sequence of *T. reesei* EGIII cellulase shown in Fig. 11 by substitution of one or more amino acid residues, said substitution being selected from: T11S/T16I, G41A, N91 D, S143T, T163S, N167S, V210C, and T145E/Y147W, said amino acid numbering being as in Figure 11; wherein the improved cellulase has increased thermostability compared to a cellulase corresponding to the precursor amino acid sequence.

2. An improved cellulase comprising an amino acid sequence which has been modified from a precursor amino acid sequence having the sequence of *H. grisea* EGIII cellulase shown in Fig. 11 by substitution of one or more amino acid residues, said substitution being selected from: C210V and C170G, said amino acid numbering being as in Figure 11; wherein the improved cellulase has increased thermostability compared to a cellulase corresponding to the precursor amino acid sequence.

3. A DNA sequence encoding a cellulase according to claim 1 or 2.

4. An expression vector comprising a DNA sequence according to claim 3.

5. A host cell comprising a DNA sequence according to claim 3 or an expression vector according to claim 4.

6. A method of producing a cellulase according to claim 1 or 2, comprising:

   (a) modifying a DNA sequence encoding the precursor amino acid sequence to produce a modified DNA sequence consisting of the sequence as defined in claim 3;
   (b) expressing the modified DNA sequence in a host cell; and
   (c) purifying the expressed cellulase protein.

7. Use of a cellulase according to claim 1 or 2 in biomass reduction, cleaning products or textile treatment compositions.

## Patentansprüche

1. Verbesserte Cellulase, umfassend eine Aminosäuresequenz, die aus einer Vorläufer-Aminosäuresequenz mit der Sequenz von der in Fig. 11 gezeigten EGIII-Cellulase aus *T. reesei* durch Substitution von einem oder mehr Aminosäurerest(en) modifiziert wurde, wobei die Substitution ausgewählt ist aus: T11S/T16I, G41A, N91D, S143T, T163S, N167S, V210C und T145E/Y147W, wobei die Aminosäure-Nummerierung wie in Figur 11 ist; wobei die verbesserte Cellulase verglichen mit einer Cellulase, die der Vorläufer-Aminosäuresequenz entspricht, eine erhöhte Thermostabilität aufweist.

2. Verbesserte Cellulase, umfassend eine Aminosäuresequenz, die aus einer Vorläufer-Aminosäuresequenz mit der Sequenz von der in Fig. 11 gezeigten EGIII-Cellulase aus *H. grisea* durch Substitution von einem oder mehr Aminosäurerest(en) modifiziert wurde, wobei die Substitution ausgewählt ist aus: C210V und C170G, wobei die Aminosäure-Nummerierung wie in Figur 11 ist; wobei die verbesserte Cellulase verglichen mit einer Cellulase, die der Vorläufer-Aminosäuresequenz entspricht, eine erhöhte Thermostabilität aufweist.

3. DNA-Sequenz, kodierend für eine Cellulase nach Anspruch 1 oder 2.

4. Expressionsvektor, umfassend eine DNA-Sequenz nach Anspruch 3.

5. Wirtszelle, umfassend eine DNA-Sequenz nach Anspruch 3 oder einen Expressionsvektor nach Anspruch 4.

6. Verfahren zur Herstellung einer Cellulase nach Anspruch 1 oder 2, umfassend:

   (a) Modifikation einer DNA-Sequenz, die für die Vorläufer-Aminosäuresequenz kodiert, zur Herstellung einer modifizierten DNA-Sequenz, die aus der nach Anspruch 3 definierten Sequenz besteht;
   (b) Expression der modifizierten DNA-Sequenz in einer Wirtszelle; und
   (c) Aufreinigung des exprimierten Cellulaseproteins.

7. Verwendung einer Cellulase nach Anspruch 1 oder 2 bei der Biomassenreduktion, in Reinigungsprodukten oder Zusammensetzungen für die Textilbehandlung.

## Revendications

1. Cellulase améliorée comprenant une séquence d'acides aminés qui a été modifiée à partir d'une séquence d'acides aminés précurseur présentant la séquence de la cellulase EGIII de *T. reesei* montrée dans la Fig. 11 par la substitution d'un ou de plusieurs résidus d'acides aminés, ladite substitution étant choisie parmi: T11S/T16I, G41A, N91D, S143T, T163S, N167S, V210C et T145E/Y147W, ladite numérotation des acides aminés étant comme dans la Figure 11; où la cellulase améliorée possède une thermostabilité augmentée comparée à une cellulase correspondant à la séquence d'acides aminés précurseur.

2. Cellulase améliorée comprenant une séquence d'acides aminés qui a été modifiée à partir d'une séquence d'acides aminés précurseur présentant la séquence de la cellulase EGIII de *H. grisea* montrée dans la Fig. 11 par la substitution d'un ou de plusieurs résidus d'acides aminés, ladite substitution étant choisie parmi: C210V et C170G, ladite numérotation des acides aminés étant comme dans la Figure 11; où la cellulase améliorée possède une thermostabilité augmentée comparée à une cellulase correspondant à la séquence d'acides aminés précurseur.

**3.** Séquence d’ADN codant pour une cellulase selon la revendication 1 ou 2.

**4.** Vecteur d’expression comprenant une séquence d’ADN selon la revendication 3.

**5.** Cellule hôte comprenant une séquence d’ADN selon la revendication 3 ou un vecteur d’expression selon la revendication 4.

**6.** Procédé pour la production d’une cellulase selon la revendication 1 ou 2, comprenant:

(a) la modification d’une séquence d’ADN codant pour la séquence d’acides aminés précurseur pour produire une séquence d’ADN modifiée constituée de la séquence telle que définie dans la revendication 3;
(b) l’expression de la séquence d’ADN modifiée dans une cellule hôte; et
(c) la purification de la protéine de cellulase exprimée.

**7.** Utilisation d’une cellulase selon la revendication 1 ou 2 dans une réduction de biomasse, des produits nettoyants ou des compositions de traitement de textiles.

188

N188T 5'-G GAT TGG GAA GTG TCG ACT GAA AAC GGC AAC TAT GAT-3' (SEQ ID NO:4)
Sall

N188P 5'-G GAT TGG GAA GTT TCC CCA GAA AAT GGC AAC TAT GAT-3' (SEQ ID NO:5)
pflMI

N188R 5'-G GAT TGG GAA GTT TCT AGA GAA AAC GGC AAC TAT GAT-3' (SEQ ID NO:6)
Xbal

N188L 5'-G GAT TGG GAA GTT TCC CTC GAG AAC GGC AAC TAT GAT-3' (SEQ ID NO:7)
Xhol

N188A 5'-G GAT TGG GAA GTT TCG GCC GAA AAC GGC AAC TAT GAT-3' (SEQ ID NO:8)
Eagl

N188G 5'-G GAT TGG GAA GTT TCC GGA GAA AAC GGC AAC TAT GAT-3' (SEQ ID NO:9)
BspEI

N188V 5'-G GAT TGG GAA GTT AGC GTC GAA AAC GGC AAC TAT GAT-3' (SEQ ID NO:10)
Hgal

N188K 5'-G GAT TGG GAA GTT TCC AAG GAA AAC GGC AAC TAT GAT-3' (SEQ ID NO:11)
Styl

N188Q 5'-G GAT TGG GAA GTT TCC CAG GAA AAT GGC AAC TAT GAT-3' (SEQ ID NO:12)
BstXI

N188H 5'-G GAT TGG GAA GTT TCT CAT GAA AAC GGC AAC TAT GAT-3' (SEQ ID NO:13)
BspHI

FIG._1A

188

N188E 5'-G GAT TGG GAA GTT TCC GAA GAG AAC GGC AAC TAT GAT-3' (SEQ ID NO:14)
EarI

N188D 5'-G GAT TGG GAA GTT TCC GAG GAG AAC GGC AAC TAT GAT-3' (SEQ ID NO:15)
BseRI

N188Y 5'-G GAT TGG GAA GTT TCA TAT GAA AAC GGC AAC TAT GAT-3' (SEQ ID NO:16)
NdeI

N188C 5'-G GAT TGG GAA GTC TCC TGC GAA AAC GGC AAC TAT GAT-3' (SEQ ID NO:17)
BsmAI

N188F 5'-G GAT TGG GAA GTT TCC TTC GAA AAC GGC AAC TAT GAT-3' (SEQ ID NO:18)
BstBI

N188I 5'-G GAT TGG GAA GTT TCG ATC GAA AAC GGC AAC TAT GAT-3' (SEQ ID NO:19)
PvuI

N188M 5'-G GAT TGG GAA GTT TCC ATG GAA AAC GGC AAC TAT GAT-3' (SEQ ID NO:20)

N188w 5'-G GAT TGG GAA GTT TCC TGG GAA AAC GGC AAC TAT GAT-3' (SEQ ID NO:21)
BstNI

N188S 5'-G GAT TGG GAA GTG AGC TCT GAA AAC GGC AAC TAT GAT-3' (SEQ ID NO:22)
SstI

FIG._1B

| | | |
|---|---|---|
| PCR A+ | 179<br>5'-AGG AAA GGC TTG GGA TTG GGA AGT-3' | (SEQ ID NO:23) |
| PCR A– | 179<br>5'-ACT TCC CAA TCC CAA GCC TTT CCT-3' | (SEQ ID NO:24) |
| PCR B+ | 191<br>5'-GGC AAC TAT GAT TAT TTG ATG TAT-3' | (SEQ ID NO:25) |
| PCR B– | 191<br>5'-ATA CAT CAA ATA ATC ATA GTT GCC-3' | (SEQ ID NO:26) |
| PCR LAAfs5 | 90<br>5'-CTT CAT TCC CGC GAC ATT AAC-3' | (SEQ ID NO:27) |
| PCR ClaI-SalI | 356<br>5'-GA TTC CCT TGT GAG AAT AAA AG-3' | (SEQ ID NO:28) |
| PCR I+ | 246<br>5'-AAT CAT GTC AGG GAA AAA ACT GGG-3'<br>BsrI | (SEQ ID NO:29) |
| PCR I– | 246<br>5'-CCC AGT TTT TTC CCT GAC ATG ATT-3'<br>BsrI | (SEQ ID NO:30) |
| PCR J+ | 257<br>5'-TTT ACG GTA GCT GAA TAT TGG CAG-3' | (SEQ ID NO:31) |
| PCR J– | 257<br>5'-CTG CCA ATA TTC AGC TAC CGT AAA-3' | (SEQ ID NO:32) |

FIG._2

```
AGCTTGAAGA AGTGAAGAAG CAGAGAGGCT ATTGAATAAA TGAGTAGAAA GCGCCATATC     60

GGCGCTTTTC TTTTGGAAGA AAATATAGGG AAAATGGTAC TTGTTAAAAA TTCGGAATAT    120

TTATACAACA TCATATGTTT CACATTGAAA GGGGAGGAGA ATCATGAAAC AACAAAAACG    180
                                               M  K  Q   Q  K  R

GCTTTACGCC CGATTGCTGA CGCTGTTATT TGCGCTCATC TTCTTGCTGC CTCATTCTGC    240
 L  Y  A   R  L  L  T   L  L  F   A  L  I   F  L  L  P   H  S  A

AGCAGCGGCG GCAAATCTTA ATGGGACGCT GATGCAGTAT TTTGAATGGT ACATGCCCAA    300
 A  A  A   A  N  L  N   G  T  L   M  Q  Y   F  E  W  Y   M  P  N

TGACGGCCAA CATTGGAAGC GTTTGCAAAA CGACTCGGCA TATTTGGCTG AACACGGTAT    360
 D  G  Q   H  W  K  R   L  Q  N   D  S  A   Y  L  A  E   H  G  I

TACTGCCGTC TGGATTCCCC CGGCATATAA GGGAACGAGC CAAGCGGATG TGGGCTACGG    420
 T  A  V   W  I  P  P   A  Y  K   G  T  S   Q  A  D  V   G  Y  G

TGCTTACGAC CTTTATGATT TAGGGGAGTT TCATCAAAAA GGGACGGTTC GGACAAAGTA    480
 A  Y  D   L  Y  D  L   G  E  F   H  Q  K   G  T  V  R   T  K  Y

CGGCACAAAA GGAGAGCTGC AATCTGCGAT CAAAAGTCTT CATTCCCGCG ACATTAACGT    540
 G  T  K   G  E  L  Q   S  A  I   K  S  L   H  S  R  D   I  N  V

TTACGGGGAT GTGGTCATCA ACCACAAAGG CGGCGCTGAT GCGACCGAAG ATGTAACCGC    600
 Y  G  D   V  V  I  N   H  K  G   G  A  D   A  T  E  D   V  T  A

GGTTGAAGTC GATCCCGCTG ACCGCAACCG CGTAATTTCA GGAGAACACC TAATTAAAGC    660
 V  E  V   D  P  A  D   R  N  R   V  I  S   G  E  H  L   I  K  A
```

FIG._3A

```
CTGGACACAT TTTCATTTTC CGGGGCGCGG CAGCACATAC AGCGATTTTA AATGGCATTG   720
 W  T  H   F  H  F  P   G  R  G   S  T  Y   S  D  F  K   W  H  W

GTACCATTTT GACGGAACCG ATTGGACGA GTCCCGAAAG CTGAACCGCA TCTATAAGTT    780
 Y  H  F   D  G  T  D   W  D  E   S  R  K   L  N  R  I   Y  K  F

TCAAGGAAAG GCTTGGGATT GGGAAGTTTC CAATGAAAAC GGCAACTATG ATTATTTGAT   840
 Q  G  K   A  W  D  W   E  V  S   N  E  N   G  N  Y  D   Y  L  M

GTATGCCGAC ATCGATTATG ACCATCCTGA TGTCGCAGCA GAAATTAAGA GATGGGGCAC   900
 Y  A  D   I  D  Y  D   H  P  D   V  A  A   E  I  K  R   W  G  T

TTGGTATGCC AATGAACTGC AATTGGACGG TTTCCGTCTT GATGCTGTCA AACACATTAA   960
 W  Y  A   N  E  L  Q   L  D  G   F  R  L   D  A  V  K   H  I  K

ATTTTCTTTT TTGCGGGATT GGGTTAATCA TGTCAGGGAA AAAACGGGGA AGGAAATGTT  1020
 F  S  F   L  R  D  W   V  N  H   V  R  E   K  T  G  K   E  M  F

TACGGTAGCT GAATATTGGC AGAATGACTT GGGCGCGCTG GAAAACTATT TGAACAAAAC  1080
 T  V  A   E  Y  W  Q   N  D  L   G  A  L   E  N  Y  L   N  K  T

AAATTTTAAT CATTCAGTGT TTGACGTGCC GCTTCATTAT CAGTTCCATG CTGCATCGAC  1140
 N  F  N   H  S  V  F   D  V  P   L  H  Y   Q  F  H  A   A  S  T

ACAGGGAGGC GGCTATGATA TGAGGAAATT GCTGAACGGT ACGGTCGTTT CCAAGCATCC  1200
 Q  G  G   G  Y  D  M   R  K  L   L  N  G   T  V  V  S   K  H  P

GTTGAAATCG GTTACATTTG TCGATAACCA TGATACACAG CCGGGGCAAT CGCTTGAGTC  1260
 L  K  S   V  T  F  V   D  N  H   D  T  Q   P  G  Q  S   L  E  S

GACTGTCCAA ACATGGTTTA AGCCGCTTGC TTACGCTTTT ATTCTCACAA GGGAATCTGG  1320
 T  V  Q   T  W  F  K   P  L  A   Y  A  F   I  L  T  R   E  S  G
```

FIG._3B

```
ATACCCTCAG GTTTTCTACG GGGATATGTA CGGGACGAAA GGAGACTCCC AGCGCGAAAT    1380
 Y  P  Q   V  F  Y  G   D  M  Y   G  T  K   G  D  S  Q   R  E  I

TCCTGCCTTG AAACACAAAA TTGAACCGAT CTTAAAAGCG AGAAAACAGT ATGCGTACGG    1440
 P  A  L   K  H  K  I   E  P  I   L  K  A   R  K  Q  Y   A  Y  G

AGCACAGCAT GATTATTTCG ACCACCATGA CATTGTCGGC TGGACAAGGG AAGGCGACAG    1500
 A  Q  H   D  Y  F  D   H  H  D   I  V  G   W  T  R  E   G  D  S

CTCGGTTGCA AATTCAGGTT TGGCGGCATT AATAACAGAC GGACCCGGTG GGGCAAAGCG    1560
 S  V  A   N  S  G  L   A  A  L   I  T  D   G  P  G  G   A  K  R

AATGTATGTC GGCCGGCAAA ACGCCGGTGA GACATGGCAT GACATTACCG GAAACCGTTC    1620
 M  Y  V   G  R  Q  N   A  G  E   T  W  H   D  I  T  G   N  R  S

GGAGCCGGTT GTCATCAATT CGGAAGGCTG GGGAGAGTTT CACGTAAACG GCGGGTCGGT    1680
 E  P  V   V  I  N  S   E  G  W   G  E  F   H  V  N  G   G  S  V

TTCAATTTAT GTTCAAAGAT AGAAGAGCAG AGAGGACGGA TTTCCTGAAG GAAATCCGTT    1740
 S  I  Y   V  Q  R  *

TTTTTATTTT GCCCGTCTTA TAAATTTCTT TGATTACATT TTATAATTAA TTTTAACAAA    1800

GTGTCATCAG CCCTCAGGAA GGACTTGCTG ACAGTTTGAA TCGCATAGGT AAGGCGGGGA    1860

TGAAATGGCA ACGTTATCTG ATGTAGCAAA GAAAGCAAAT GTGTCGAAAA TGACGGTATC    1920

GCGGGTGATC AATCATCCTG AGACTGTGAC GGATGAATTG AAAAAGCT                 1968
```

FIG._3C

```
ANLNGTLMQY FEWYMPNDGQ HWKRLQNDSA YLAEHGITAV WIPPAYKGTS QADVGYGAYD   60
LYDLGEFHQK GTVRTKYGTK GELQSAIKSL HSRDINVYGD VVINHKGGAD ATEDVTAVEV  120
DPADRNRVIS GEHLIKAWTH FHFPGRGSTY SDFKWHWYHF DGTDWDESRK LNRIYKFQGK  180
AWDWEVSNEN GNYDYLMYAD IDYDHPDVAA EIKRWGTWYA NELQLDGFRL DAVKHIKFSF  240
LRDWVNHVRE KTGKEMFTVA EYWQNDLGAL ENYLNKTNFN HSVFDVPLHY QFHAASTQGG  300
GYDMRKLLNG TVVSKHPLKS VIFVDNHDTQ PGQSLESTVQ TWFKPLAYAF ILTRESGYPQ  360
VFYGDMYGTK GDSQREIPAL KHKIEPILKA RKQYAYGAQH DYFDHHDIVG WTREGDSSVA  420
NSGLAALITD GPGGAKRMYV GRQNAGETWH DITGNRSEPV VINSEGWGEF HVNGGSVSIY  480
VQR
```

FIG._4

Am-Lich = *B.licheniformis* Am-Amylo = *B.amyloliquefaciens* Am-Stero = *B.stearothermophilus.*

```
                                                           1            19
           1                                                            60
Am-Lich    ......MKQQ KRLYARLLTL LFALIFLLPH ......SAAA AANLNGTLMQ YFEWYMPNDG
Am-Amylo   MRGRGNMIQK RKRTVSFRLV LMCTLLFVSL ......PITK TSAVNGTLMQ YFEWYTPNDG
Am-Stearo  ......VLTF HRIIRKGWMF LLAFLLTASL FCPTGRHAKA AAPFNGTMMQ YFEWYLPDDG

                                                                        79
           61                                                          120
Am-Lich    QHWKRLQNDS AYLAEHGITA VWIPPAYKGT SQADVGYGAY DLYDLGEFHQ KGTVRTKYGT
Am-Amylo   QHWKRLQNDA EHLSDIGITA VWIPPAYKGL SQSDNGYGPY DLYDLGEFQQ KGTVRTKYGT
Am-Stearo  TLWTKVANEA NNLSSLGITA LSLPPAYKGT SRSDVGYGVY DLYDLGEFNQ KGTVRTKYGT

                                                                       139
           121                                                         180
Am-Lich    KGELQSAIKS LHSRDINVYG DVVINHKGGA DATEDVTAVE VDPADRNRVI SGEHLIKAWT
Am-Amylo   KSELQDAIGS LHSRNVQVYG DVVLNHKAGA DATEDVTAVE VNPANRNQET SEEYQIKAWT
Am-Stearo  KAQYLQAIQA AHAAGMQVYA DVVFDHKGGA DGTEWVDAVE VNPSDRNQEI SGTYQIQAWT

                                                                       197
           181                                                         240
Am-Lich    HFHFPGRGST YSDFKWHWYH FDGTDWDESR KLNRIYKF.. QGKAWDWEVS NENGNYDYLM
Am-Amylo   DFRFPGRGNT YSDFKWHWYH FDGADWDESR KISRIFKFRG EGKAWDWEVS SENGNYDYLM
Am-Stearo  KFDFPGRGNT YSSFKWRWYH FDGVDWDESR KLSRIYKFRG IGKAWDWEVD TENGNYDYLM

                                                                       257
           241                                                         300
Am-Lich    YADIDYDHPD VAAEIKRWGT WYANELQLDG FRLDAVKHIK FSFLRDWVNH VREKTGKEMF
Am-Amylo   YADVDYDHPD VVAETKKWGI WYANELSLDG FRIDAAKHIK FSFLRDWVQA VRQATGKEMF
Am-Stearo  YADLDMDHPE VVTELKNWGK WYVNTTNIDG FRLDGLKHIK FSFFPDWLSY VRSQTGKPLF
```

FIG._5A

```
Am-Lich    = B.licheniformis    Am-Amylo = B.amyloliquefaciens    Am-Stero = B.stearothermophilus.

                                                                           317
           301                                                             360
Am-Lich    TVAEYWQNDL GALENYLNKT NFNHSVFDVP LHYQFHAAST QGGGYDMRKL LNGTVVSKHP
Am-Amylo   TVAEYWQNNA GKLENYLNKT SFNQSVFDVP LHFNLQAASS QGGGYDMRRL LDGTVVSRHP
Am-Stearo  TVGEYWSYDI NKLHNYITKT NGTMSLFDAP LHNKFYTASK SGGAFDMRTL MTNTLMKDQP

                                                                           377
           361                                                             420
Am-Lich    LKSVTFVDNH DTQPGQSLES TVQTWFKPLA YAFILTRESG YPQVFYGDMY GTKGDSQREI
Am-Amylo   EKAVTFVENH DTQPGQSLES TVQTWFKPLA YAFILTRESG YPQVFYGDMY GTKGTSPKEI
Am-Stearo  TLAVTFVDNH DTNPAKR.CS HGRPWFKPLA YAFILTRQEG YPCVFYGDYY GI...PQYNI

                                                                           437
           421                                                             480
Am-Lich    PALKHKIEPI LKARKQYAYG AQHDYFDHHD IVGWTREGDS SVANSGLAAL ITDGPGGAKR
Am-Amylo   PSLKDNIEPI LKARKEYAYG PQHDYIDHPD VIGWTREGDS SAAKSGLAAL ITDGPGGSKR
Am-Stearo  PSLKSKIDPL LIARRDYAYG TQHDYLDHSD IIGWTREGVT EKPGSGLAAL ITDGAGRSKW

                                                    483
           481                                                             540
Am-Lich    MYVGRQNAGE TWHDITGNRS EPVVINSEGW GEFHVNGGSV SIYVQR.... ..........
Am-Amylo   MYAGLKNAGE TWYDITGNRS DTVKIGSDGW GEFHVNDGSV SIYVQK.... ..........
Am-Stearo  MYVGKQHAGK VFYDLTGNRS DTVTINSDGW GEFKVNGGSV SVWVPRKTTV STIARPITTR

           541               559
Am-Lich    .......... .........
Am-Amylo   .......... .........
Am-Stearo  PWTGEFVRWH EPRLVAWP*
```

FIG._5B

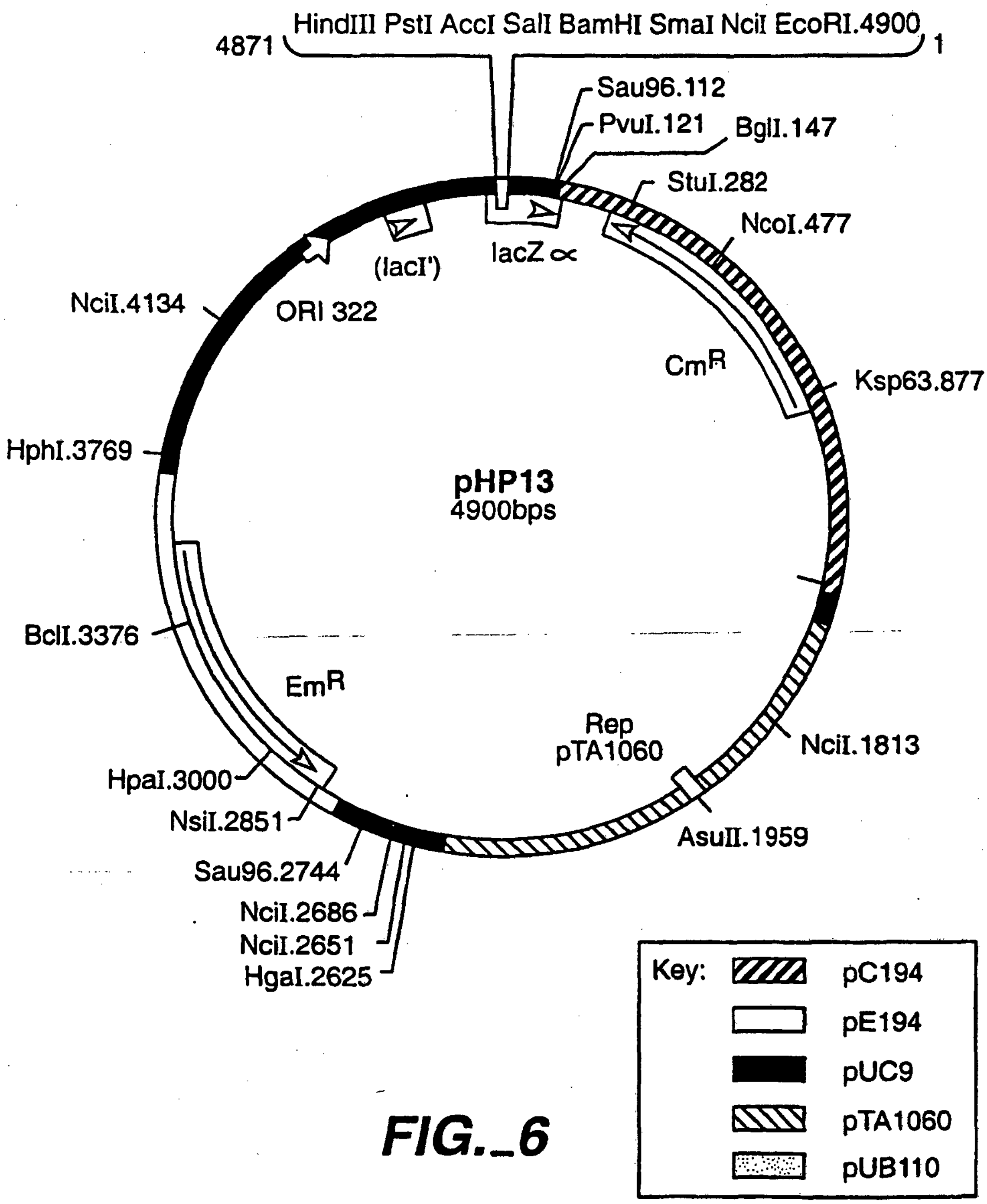
HindIII PstI AccI SalI BamHI SmaI NciI EcoRI.4900
4871
1
Sau96.112
PvuI.121
BglI.147
StuI.282
NcoI.477
lacZ ∝
(lacI')
ORI 322
NciI.4134
CmR
Ksp63.877
HphI.3769
pHP13
4900bps
BclI.3376
EmR
Rep
pTA1060
NciI.1813
HpaI.3000
NsiI.2851
AsuII.1959
Sau96.2744
NciI.2686
NciI.2651
HgaI.2625
Key:
pC194
pE194
pUC9
pTA1060
pUB110

FIG._6

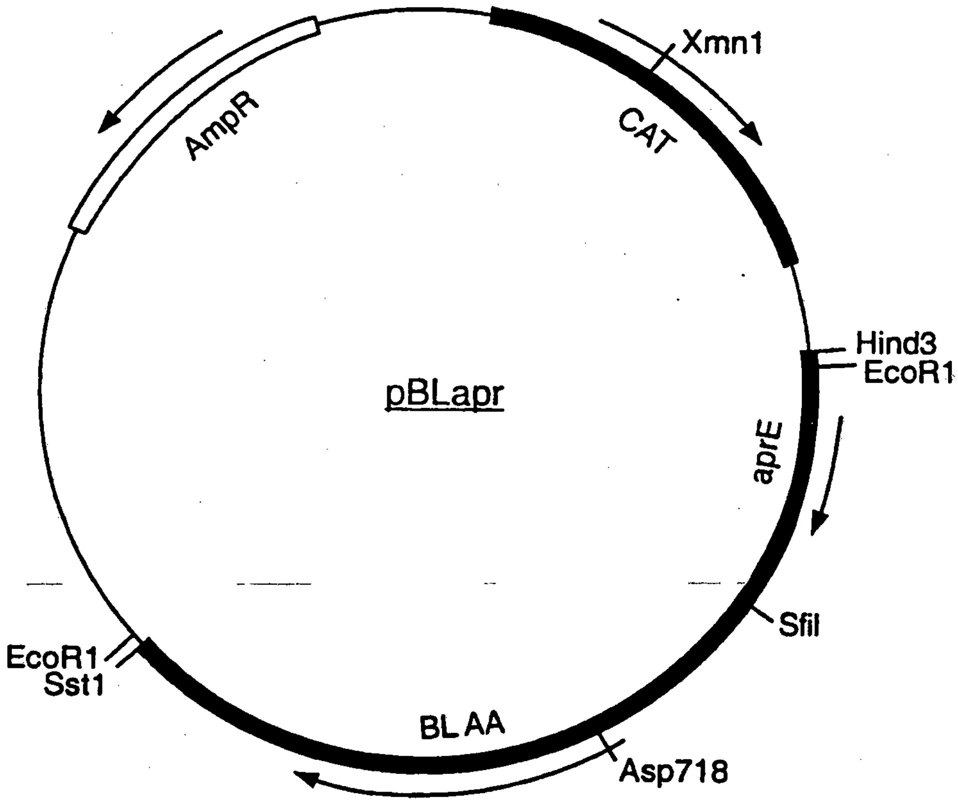
Xmn1
AmpR
CAT
Hind3
EcoR1
pBLapr
aprE
SfiI
EcoR1
Sst1
BLAA
Asp718

FIG._7

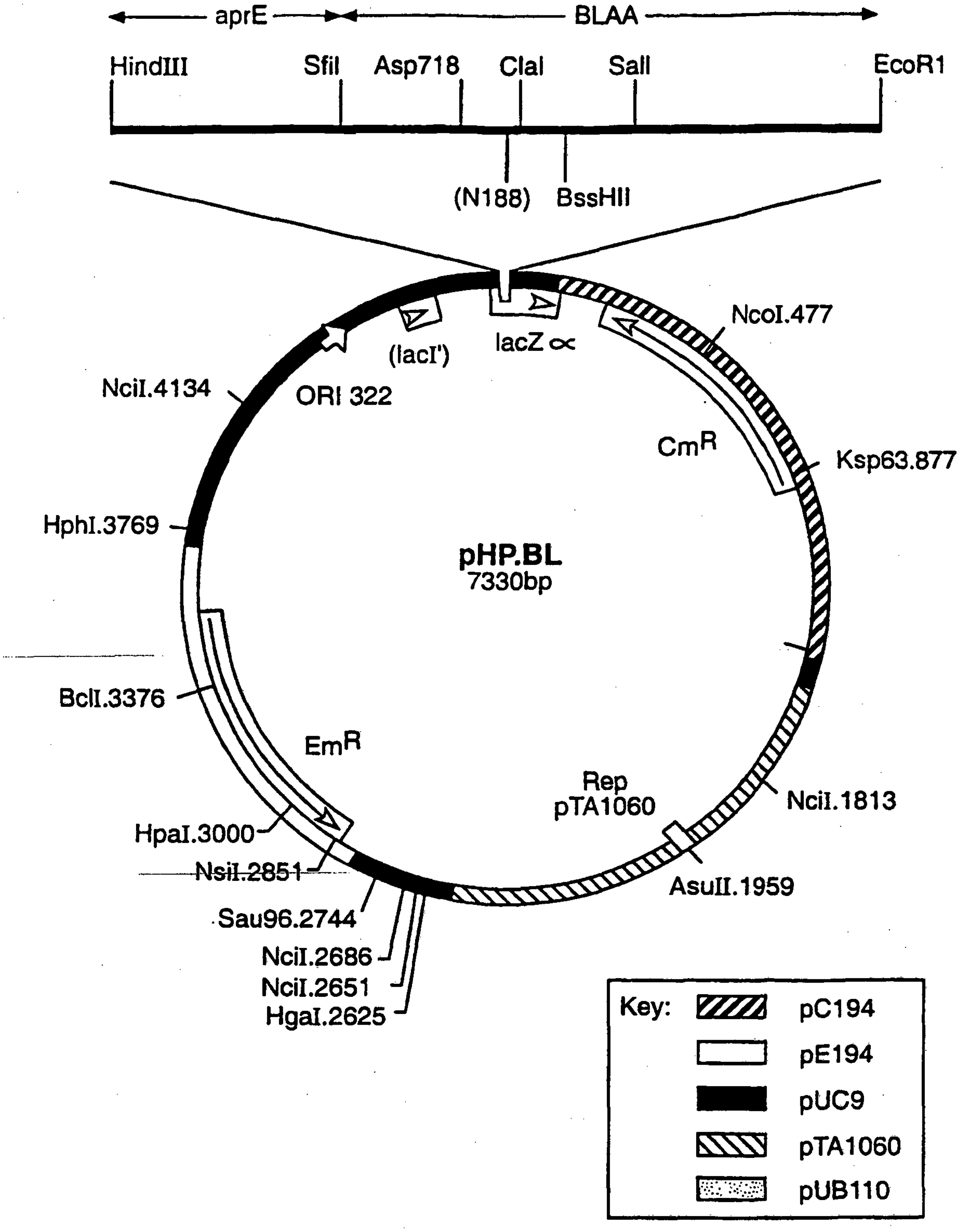


pHP.BL = pHP13 with the 2460bp HindIII-EcoRI insert from pBLapr

FIG._8

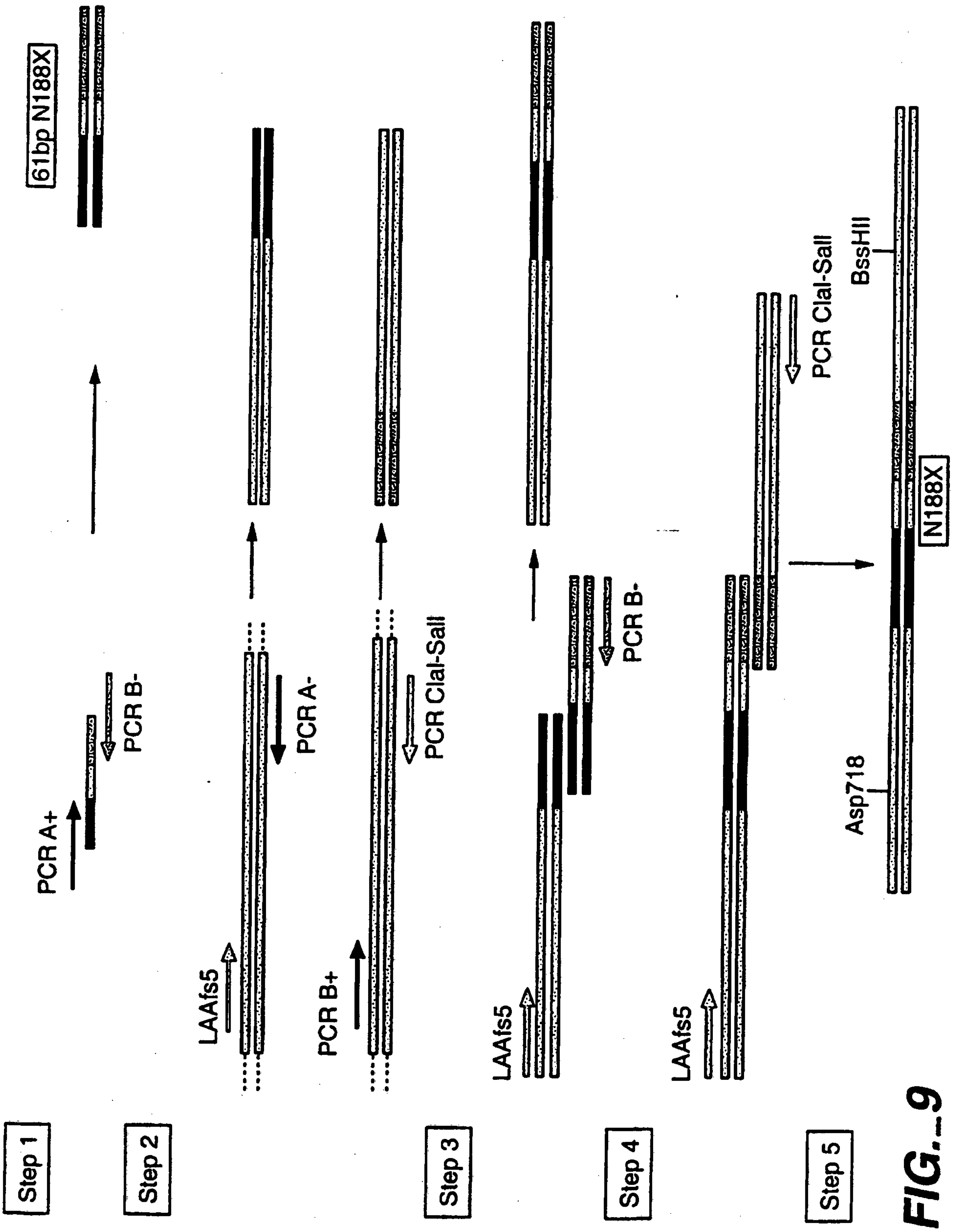
Step 1
61bp N188X
PCR A+
PCR B-
Step 2
LAAfs5
PCR A-
PCR B+
PCR ClaI-SalI
Step 3
LAAfs5
PCR B-
Step 4
LAAfs5
PCR ClaI-SalI
Step 5
Asp718
N188X
BssHII

FIG._9

SIGNAL SEQUENCE-MATURE PROTEIN JUNCTIONS IN:

*B.licheniformis* alpha-amylase.

(PstI)

M K Q Q K R L T A R L L T L L F A L I F L L P H S A A A A **A N L** . . . . . . . . . . . .

*B.subtilis* alkaline protease aprE.

(PstI)

*M R S K T L W I S L L F A L T L I F T M A F S N M S A Q A* **A G K S** . . . . . . . . . . .

*B.licheniformis* alpha-amylase in pBLapr.

*M R S K T L W I S L L F A L T L I F T M A F S N M S A Q A* **A N L** . . . . . . . . . . . .

(PstI) ↓ indicates the site of the restriction site in the gene

**BOLD TYPE** indicates the N-terminus of the secreted protein in *Bacillus*.

FIG._10

```
                                            1                                  17
        T._reesei   M.........KF.LQVLPALIPAALAQTS...............CDQWATFTGNG..YTV
  H._schweinitzii   M.........KF.LQVLPAILPAALAQTS...............CDQYATFSGNG..YIV
    A._aculeatus__* M.........KAFHL.LAALAGAAVAQQAQ.............LCDQYATYTGGV..YTI
    A._kawachii_2   M.........KAFHL.LAALSGAAVAQQAQ.............LCDQYATYTGGV..YTI
        H._grisei   M........LKSALLLGAAAVSVQSASIPTIPANLEPRQIR.SLCELYGYWSGNG..YEL
   G._roseum_Rj_1   M.........KANIVILSLFAPLAAVAQT..............LCGQYSSNTQGG..YIF
   G._roseum_PA_3   M.........KFQLLSLTAFAPLSLAA................LCGQYQSQSQGG..YIF
   G._roseum_Rj_4   M.........KTGIAYLAAVLPLA.MAES..............LCDQYAYLSRDG..YNF
                    18                                               63
        T._reesei   SNNLWGASAGSGF..GCV.TAVSLSGG.ASWHADWQWSGGQNNVKSYQNS..........
  H._schweinitzii   SNNLWGASAGSGF..GCV.TSVSLNGA.ASWHADWQWSGGQNNVKSYQNV..........
    A._aculeatus__* NNNLWGKDAGSG..SQCTTVNSASSAG.TSWSTKWNWSGGENSVKSYANS..........
    A._kawachii_2   NNNLWGKDAGSG..SQCTTVNSASSAG.TSWSTKWNWSGGENSVKSYANS..........
        H._grisei   LNNLWGKDTATS.GWQCTYLDGTNNGG.IQWNTAWEWQGAPDNVKNYPYV..........
   G._roseum_Rj_1   NNNMWGMGSGSGS..QCTYVDKVWAEG.VAWHTDWSWSGGDNNVKSYPYS..........
   G._roseum_PA_3   NNNKWGQGSGSGS..QCLTIDKTWDSN.VAFHADWSWSGGTNNVKSYPNA..........
   G._roseum_Rj_4   NNNEWGAATGTGD..QCTYVDSTSSGG.VSWHSDWTWSGSESEIKSYPYS..........
                    64                                                          117
        T._reesei   .QIAIP.QKRTVNSISSMPTTASW...SYSGSNIRANVAYDL.FTAANPNHVTYSGDYEL
  H._schweinitzii   .QINIP.QKRTVNSIGSMPTTASW...SYSGSDIRANVAYDL.FTAANPNHVTYSGDYEL
    A._aculeatus__* .GLTF..NKKLVSQISQIPTTARW.S..YDNTGIRADVAYDL.FTAADINHVTWSGDYEL
    A._kawachii_2   .GLSF..NKKLVSQISHIPTAARW.S..YDNTCIRRGRAYDL.FTAADINHVTWSGDYEL
        H._grisei   .GKQIQRGRK.ISDINSMRTSVSW...TYDRTDLRANVAYDV.FTARDPDHPNWGGDYEL
   G._roseum_Rj_1   .GRELGT.KRIVSSIKSISSGADW...DYTGSNLRANAAYDI.FTSANPNHATSSGDYEV
   G._roseum_PA_3   .GLEFSR.GKKVSSIGTINGGADW...DYSGSNIRANVAYGI.FTSADPNHVTSSGDYEL
   G._roseum_Rj_4   .GLDLPE.KKIVTSIGSISTGAEW...SYSGSDIRADVAYDT.FTAADPNHATSSGDYEV
```

FIG._11A

```
                    118                                                          166
T._reesei           MIWLGKYGDIGPIGSS....QGTVNVGGQSWTLYYGYNGAMQV......YSFVAQT.NTT
H._schweinitzii     MIWLGKYGDIGPIGSS....QGTVNVGGQTWTLYYGYNGAMQV......YSFVAQS.NTT
A._aculeatus__*     MIWLARYGGVQPIGSQ....IATATVDGQTWELWYG......ANGSQKTYSFVAPT.PIT
A._kawachii_2       MIWLARYGGVQPLGSQ....IATATVEGQTWELWYG......VNGAQKTYSFVAAN.PIT
H._grisei           MIWLARYGGIYPIGTF....HSQVNLAGRTWDLWTGYNGNMRV......YSFLPPSGDIR
G._roseum_Rj_1      MIWLANLGGLTPIGSP....IGTVKAAGRDWELWDGYNGAMRV......YSFVAPS.QLN
G._roseum_PA_3      MIWLGKLGDIYPIGNS....IGRVEAANREWDFLVGYNGAMKV......FSFVAPS.PVT
G._roseum_Rj_4      MIWLANLGGLTPIGSP....IGTVKAAGRDWELWDGYNGAMRV......YSFVAPS.QLN

                    167                                                          218
T._reesei           NYSGDVKNFFNYLRDNKGYNAAGQYV..LSYQFGTEPF..TGSGT.LNVASWTASI.N..
H._schweinitzii     SYSGDVKNFFNYLRDNKGYNAGGQYV..LSYQFGTEPF..TGSGT.LNVASWTASI.N..
A._aculeatus        SFQGDVNDFFKYLTQNHGFPASSQYLI..TLQFGTEPF..TGGPATLSVSNWSASVQQAG
A._kawachii_2       SFQGDINDFFKYLTQNHGFPASSQYLIILALQFGTEPF..TGGPATLNVADWSASVQ...
H._grisei           DFSCDIKDFFNYLERNHGYPAREQNLIV..YQVGTECF..TGGPARFTCRDFRADL....
G._roseum_Rj_1      SFDGEIMDFFYVVKDMRGFPADSQHL..LTVQFGTEPI..SGSGAKFSVSHWSAKLG...
G._roseum_PA_3      LFDGNIMDFFYVMRDMQGYPMDKQYL..LSLQFGTEPF..TGSNANFSCWYFGAKIK...
G._roseum_Rj_4      SFDGEIMDFFYVVKDMRGFPADSQHL..LTVQFGTEPI..SGSGAKFSVSHWSAKLG...

A._aculeatus        EPWQNGAGLAVNSFSSTV
H._grisei           ..W
```

FIG._11B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4760025 A
- US 4752585 A
- US 4732973 A
- US 5605793 A, Stemmer
- WO 0014206 A2
- WO 9931255 A2
- WO 9524471 A1
- US 5185258 A
- WO 9623874 A, PCT
- US 194664 A
- WO 9418314 A
- US 5264366 A, Ferrari
- EP 00984363 A
- WO 00984363 A
- US 0033878 W
- US 09470832 B


### Non-patent literature cited in the description


- **SIEZEN et al.** *Protein Engineering,* 1991, vol. 4 (7), 719-737
- **GRAY et al.** *J. Bacteriology,* 1986, vol. 166, 635-643
- **TAKKINEN et al.** *J. Biol. Chem.,* 1983, vol. 258, 1007-1013
- **IHARA et al.** *Biochem.,* 1985, vol. 98, 95-103
- **NAKAJIMA et al.** *Appl. Microbiol. Biotechnol.,* 1986, vol. 23, 355-360
- **ROGERS.** *Biochem. Biophys. Res. Commun.,* 1985, vol. 128, 470-476
- **JANECEK.** *Eur. J. Biochem.,* 1994, vol. 224, 519-524
- **FENG et al.** *J. Molec. Evol.,* 1987, vol. 35, 351-360
- **HOLM et al.** *Protein Engineering,* 1990, vol. 3 (3), 181-191
- **BUISSON et al.** *EMBO Journal,* 1987, vol. 6, 3909-3916
- **QIAN et al.** *Biochemistry,* 1994, vol. 33, 6284-6294
- **LARSON et al.** *J. Mol. Biol.,* 1994, vol. 235, 1560-1584
- **MATSUURA et al.** *J. Biochem. (Tokyo),* 1984, vol. 95, 697-702
- **BOEL et al.** *Biochemistry,* 1990, vol. 29, 6244-6249
- **VALLEE et al.** *J. Mol. Biol.,* 1994, vol. 236, 368-371
- **KADZIOLA.** *J. Mol. Biol.,* 1994, vol. 239, 104-121
- **SUZUKI et al.** *J. Biochem.,* 1990, vol. 108, 379-381
- **LEE et al.** *Arch. Biochem. Biophys,* 1991, vol. 291, 255-257
- **CHANG et al.** *J. Mol. Biol.,* 1993, vol. 229, 235-238
- **MIZUNO et al.** *J. Mol. Biol.,* 1993, vol. 234, 1282-1283
- **MACHIUS et al.** *J. Mol. Biol.,* 1995, vol. 246, 545-549
- **MACGREGOR et al.** *Biochem. J.,* 1989, vol. 259, 145-152
- **JASPERSEN.** *J. Prot. Chem.,* 1993, vol. 12, 791-805
- **MACGREGOR.** *Starke,* 1993, vol. 45, 232-237
- **JANECEK.** *FEBS Letters,* 1993, vol. 316, 23-26
- **JANECEK et al.** *J. Prot. Chem.,* 1993, vol. 12, 509-514
- **HOLM et al.** *Protein Engineering,* 1990, vol. 3, 181-191
- **VIHINEN et al.** *J. Biochem.,* 1990, vol. 107, 267-272
- **DALE, J.W.** Molecular Genetics of Bacteria. John Wiley & Sons, 1989
- **WELLS et al.** *Nucleic Acid Research,* 1983, vol. 11, 7911-7925
- **HAIMA et al.** *Mol. Gen. Genet.,* 1987, vol. 209, 335-342
- **SANGER et al.** *Proc. Natl. Acad. Sci. U.S.A.,* 1977, vol. 74, 5463-5467
- **ANAGNOSTOPOULOS et al.** *J. Bacter.,* 1961, vol. 81, 741-746
- **STAHL et al.** *J. Bacter.,* 1984, vol. 158, 411-418
- **HENNER et al.** *J. Bacter.,* 1988, vol. 170, 296-300
- **HOCH.** *J. Bact.,* 1983, vol. 154, 1513-1515
- **BRADFORD.** *Anal. Biochem.,* 1976, vol. 72, 248

SEQUENCE LISTING

<110> Genencor International, Inc.

<120> METHOD FOR OBTAINING PROTEINS HAVING IMPROVED FUNCTIONAL CHARACTERISTICS

<130> CSC/FP6654487

<140> Divisional application based on EP 00984363.2
<141> 2000-12-14

<150> 00984363.2 <151> 2000-12-14

<150> PCT/US00/33878

<151> 2000-12-14

<150> US 09/470,832
<151> 1999-12-23

<160> 48

<170> FastSEQ for Windows version 4.0

<210> 1
<211> 56
<212> DNA
<213> Artificial Sequence

<220>
<223> oligonucleotide

<400> 1
gatcaaaaca taaaaaaccg gccttggccc cgccggtttt ttattatttt tgagct 56

<210> 2
<211> 48
<212> DNA
<213> Artificial Sequence

<220>
<223> oligonucleotide

<400> 2
caaaaataat aaaaaaccgg cggggccaag gccggttttt tatgtttt 48

<210> 3
<211> 38
<212> DNA
<213> Artificial Sequence

<220>
<223> oligonucleotide

<400> 3
cccattaaga ttggccgcct gggccgacat gttgctgg 38

<210> 4
<211> 37
<212> DNA
<213> Artificial Sequence

<220>
<223> oligonucleotide

<400> 4
ggattgggaa gtgtcgactg aaaacggcaa ctatgat 37

<210> 5
<211> 37
<212> DNA
<213> Artificial Sequence

<220>
<223> oligonucleotide

<400> 5
ggattgggaa gtttccccag aaaatggcaa ctatgat 37

<210> 6
<211> 37
<212> DNA
<213> Artificial Sequence

<220>
<223> oligonucleotide

<400> 6
ggattgggaa gtttctagag aaaacggcaa ctatgat 37

<210> 7
<211> 37
<212> DNA
<213> Artificial Sequence

<220>
<223> oligonucleotide

<400> 7
ggattgggaa gtttccctcg agaacggcaa ctatgat 37

<210> 8
<211> 37
<212> DNA
<213> Artificial Sequence

<220>
<223> oligonucleotide

<400> 8
ggattgggaa gtttcggccg aaaacggcaa ctatgat 37

<210> 9
<211> 37
<212> DNA
<213> Artificial Sequence

<220>
<223> oligonucleotide

<400> 9
ggattgggaa gtttccggag aaaacggcaa ctatgat 37

<210> 10
<211> 37
<212> DNA
<213> Artificial Sequence

<220>
<223> oligonucleotide

<400> 10
ggattgggaa gttagcgtcg aaaacggcaa ctatgat 37

<210> 11
<211> 37
<212> DNA
<213> Artificial Sequence

<220>
<223> oligonucleotide

<400> 11
ggattgggaa gtttccaagg aaaacggcaa ctatgat 37

<210> 12
<211> 37
<212> DNA
<213> Artificial Sequence

<220>
<223> oligonucleotide

<400> 12
ggattgggaa gtttcccagg aaaatggcaa ctatgat 37

<210> 13
<211> 37
<212> DNA
<213> Artificial Sequence

<220>
<223> oligonucleotide

<400> 13
ggattgggaa gtttctcatg aaaacggcaa ctatgat 37

<210> 14
<211> 37
<212> DNA
<213> Artificial Sequence

<220>
<223> oligonucleotide

<400> 14
ggattgggaa gtttccgaag agaacggcaa ctatgat 37

<210> 15
<211> 37
<212> DNA
<213> Artificial Sequence

<220>
<223> oligonucleotide

<400> 15
ggattgggaa gtttccgagg agaacggcaa ctatgat 37

<210> 16
<211> 37
<212> DNA
<213> Artificial Sequence

<220>
<223> oligonucleotide

<400> 16
ggattgggaa gtttcatatg aaaacggcaa ctatgat 37

<210> 17
<211> 37
<212> DNA
<213> Artificial Sequence

<220>
<223> oligonucleotide

<400> 17
ggattgggaa gtctcctgcg aaaacggcaa ctatgat 37

<210> 18
<211> 37
<212> DNA
<213> Artificial Sequence

<220>
<223> oligonucleotide

<400> 18
ggattgggaa gtttccttcg aaaacggcaa ctatgat 37

<210> 19
<211> 37
<212> DNA
<213> Artificial Sequence

<220>
<223> oligonucleotide

<400> 19
ggattgggaa gtttcgatcg aaaacggcaa ctatgat 37

<210> 20
<211> 37
<212> DNA
<213> Artificial Sequence

<220>
<223> oligonucleotide

<400> 20
ggattgggaa gtttccatgg aaaacggcaa ctatgat 37

<210> 21
<211> 37
<212> DNA

<213> Artificial Sequence

<220>
<223> oligonucleotide

<400> 21
ggattgggaa gtttcctggg aaaacggcaa ctatgat 37

<210> 22
<211> 37
<212> DNA
<213> Artificial Sequence

<220>
<223> oligonucleotide

<400> 22
ggattgggaa gtgagctctg aaaacggcaa ctatgat 37

<210> 23
<211> 24
<212> DNA
<213> Artificial Sequence

<220>
<223> primer

<400> 23
aggaaaggct tgggattggg aagt 24

<210> 24
<211> 24
<212> DNA
<213> Artificial Sequence

<220>
<223> primer

<400> 24
acttcccaat cccaagcctt tcct 24

<210> 25
<211> 24
<212> DNA
<213> Artificial Sequence

<220>
<223> primer

<400> 25
ggcaactatg attatttgat gtat 24

<210> 26
<211> 24
<212> DNA
<213> Artificial Sequence

<220>

<223> primer

<400> 26
atacatcaaa taatcatagt tgcc 24

<210> 27
<211> 21
<212> DNA
<213> Artificial Sequence

<220>
<223> primer

<400> 27
cttcattccc gcgacattaa c 21

<210> 28
<211> 22
<212> DNA
<213> Artificial Sequence

<220>
<223> primer

<400> 28
gattcccttg tgagaataaa ag 22

<210> 29
<211> 24
<212> DNA
<213> Artificial Sequence

<220>
<223> primer

<400> 29
aatcatgtca gggaaaaaac tggg 24

<210> 30
<211> 24
<212> DNA
<213> Artificial Sequence

<220>
<223> primer

<400> 30
cccagttttt tccctgacat gatt 24

<210> 31
<211> 24
<212> DNA
<213> Artificial Sequence

<220>
<223> primer

<400> 31

tttacggtag ctgaatattg gcag 24

<210> 32
<211> 24
<212> DNA
<213> Artificial Sequence

<220>
<223> primer

<400> 32
ctgccaatat tcagctaccg taaa 24

<210> 33
<211> 1968
<212> DNA
<213> Bacillus licheniformis

<400> 33

```
agcttgaaga agtgaagaag cagagaggct attgaataaa tgagtagaaa gcgccatatc      60
ggcgcttttc ttttggaaga aaatataggg aaaatggtac ttgttaaaaa ttcggaatat     120
ttatacaaca tcatatgttt cacattgaaa ggggaggaga atcatgaaac aacaaaaacg     180
gctttacgcc cgattgctga cgctgttatt tgcgctcatc ttcttgctgc ctcattctgc     240
agcagcggcg gcaaatctta atgggacgct gatgcagtat tttgaatggt acatgcccaa     300
tgacggccaa cattggaagc gtttgcaaaa cgactcggca tatttggctg aacacggtat     360
tactgccgtc tggattcccc cggcatataa gggaacgagc caagcggatg tgggctacgg     420
tgcttacgac ctttatgatt taggggagtt tcatcaaaaa gggacggttc ggacaaagta     480
cggcacaaaa ggagagctgc aatctgcgat caaaagtctt cattcccgcg acattaacgt     540
ttacggggat gtggtcatca accacaaagg cggcgctgat gcgaccgaag atgtaaccgc     600
ggttgaagtc gatcccgctg accgcaaccg cgtaatttca ggagaacacc taattaaagc     660
ctggacacat tttcattttc cggggcgcgg cagcacatac agcgatttta aatggcattg     720
gtaccatttt gacggaaccg attgggacga gtcccgaaag ctgaaccgca tctataagtt     780
tcaaggaaag gcttgggatt gggaagtttc caatgaaaac ggcaactatg attatttgat     840
gtatgccgac atcgattatg accatcctga tgtcgcagca gaaattaaga gatggggcac     900
ttggtatgcc aatgaactgc aattggacgg tttccgtctt gatgctgtca aacacattaa     960
atttcttttt ttgcgggatt gggttaatca tgtcagggaa aaaacgggga aggaaatgtt    1020
tacggtagct gaatattggc agaatgactt gggcgcgctg gaaaactatt tgaacaaaac    1080
aaattttaat cattcagtgt ttgacgtgcc gcttcattat cagttccatg ctgcatcgac    1140
acagggaggc ggctatgata tgaggaaatt gctgaacggt acggtcgttt ccaagcatcc    1200
gttgaaatcg gttacatttg tcgataacca tgatacacag ccggggcaat cgcttgagtc    1260
gactgtccaa acatggttta agccgcttgc ttacgctttt attctcacaa gggaatctgg    1320
ataccctcag gttttctacg gggatatgta cgggacgaaa ggagactccc agcgcgaaat    1380
tcctgccttg aaacacaaaa ttgaaccgat cttaaaagcg agaaaacagt atgcgtacgg    1440
agcacagcat gattatttcg accaccatga cattgtcggc tggacaaggg aaggcgacag    1500
ctcggttgca aattcaggtt tggcggcatt aataacagac ggacccggtg ggcaaagcg     1560
aatgtatgtc ggccggcaaa acgccggtga gacatggcat gacattaccg gaaaccgttc    1620
ggagccggtt gtcatcaatt cggaaggctg gggagagttt cacgtaaacg gcgggtcggt    1680
ttcaatttat gttcaaagat agaagagcag agaggacgga tttcctgaag gaaatccgtt    1740

tttttatttt gcccgtctta taaatttctt tgattacatt ttataattaa ttttaacaaa    1800
gtgtcatcag ccctcaggaa ggacttgctg acagtttgaa tcgcataggt aaggcgggga    1860
tgaaatggca acgttatctg atgtagcaaa gaaagcaaat gtgtcgaaaa tgacggtatc    1920
gcgggtgatc aatcatcctg agactgtgac ggatgaattg aaaaagct                 1968
```

<210> 34
<211> 483
<212> PRT
<213> Bacillus licheniformis

<400> 34

```
Ala Asn Leu Asn Gly Thr Leu Met Gln Tyr Phe Glu Trp Tyr Met Pro
1               5                   10                  15
Asn Asp Gly Gln His Trp Lys Arg Leu Gln Asn Asp Ser Ala Tyr Leu
            20                  25                  30
Ala Glu His Gly Ile Thr Ala Val Trp Ile Pro Pro Ala Tyr Lys Gly
        35                  40                  45
Thr Ser Gln Ala Asp Val Gly Tyr Gly Ala Tyr Asp Leu Tyr Asp Leu
    50                  55                  60
Gly Glu Phe His Gln Lys Gly Thr Val Arg Thr Lys Tyr Gly Thr Lys
65                  70                  75                  80
Gly Glu Leu Gln Ser Ala Ile Lys Ser Leu His Ser Arg Asp Ile Asn
                85                  90                  95
Val Tyr Gly Asp Val Val Ile Asn His Lys Gly Gly Ala Asp Ala Thr
            100                 105                 110
Glu Asp Val Thr Ala Val Glu Val Asp Pro Ala Asp Arg Asn Arg Val
        115                 120                 125
Ile Ser Gly Glu His Leu Ile Lys Ala Trp Thr His Phe His Phe Pro
    130                 135                 140
Gly Arg Gly Ser Thr Tyr Ser Asp Phe Lys Trp His Trp Tyr His Phe
145                 150                 155                 160
Asp Gly Thr Asp Trp Asp Glu Ser Arg Lys Leu Asn Arg Ile Tyr Lys
                165                 170                 175
Phe Gln Gly Lys Ala Trp Asp Trp Glu Val Ser Asn Glu Asn Gly Asn
            180                 185                 190
Tyr Asp Tyr Leu Met Tyr Ala Asp Ile Asp Tyr Asp His Pro Asp Val
        195                 200                 205
Ala Ala Glu Ile Lys Arg Trp Gly Thr Trp Tyr Ala Asn Glu Leu Gln
    210                 215                 220
Leu Asp Gly Phe Arg Leu Asp Ala Val Lys His Ile Lys Phe Ser Phe
225                 230                 235                 240
Leu Arg Asp Trp Val Asn His Val Arg Glu Lys Thr Gly Lys Glu Met
                245                 250                 255
Phe Thr Val Ala Glu Tyr Trp Gln Asn Asp Leu Gly Ala Leu Glu Asn
            260                 265                 270
Tyr Leu Asn Lys Thr Asn Phe Asn His Ser Val Phe Asp Val Pro Leu
        275                 280                 285
His Tyr Gln Phe His Ala Ala Ser Thr Gln Gly Gly Gly Tyr Asp Met
    290                 295                 300
Arg Lys Leu Leu Asn Gly Thr Val Val Ser Lys His Pro Leu Lys Ser
305                 310                 315                 320
Val Thr Phe Val Asp Asn His Asp Thr Gln Pro Gly Gln Ser Leu Glu
                325                 330                 335
Ser Thr Val Gln Thr Trp Phe Lys Pro Leu Ala Tyr Ala Phe Ile Leu
            340                 345                 350
Thr Arg Glu Ser Gly Tyr Pro Gln Val Phe Tyr Gly Asp Met Tyr Gly
        355                 360                 365
Thr Lys Gly Asp Ser Gln Arg Glu Ile Pro Ala Leu Lys His Lys Ile
    370                 375                 380
```

Glu Pro Ile Leu Lys Ala Arg Lys Gln Tyr Ala Tyr Gly Ala Gln His
385 390 395 400
Asp Tyr Phe Asp His His Asp Ile Val Gly Trp Thr Arg Glu Gly Asp
405 410 415
Ser Ser Val Ala Asn Ser Gly Leu Ala Ala Leu Ile Thr Asp Gly Pro
420 425 430
Gly Gly Ala Lys Arg Met Tyr Val Gly Arg Gln Asn Ala Gly Glu Thr
435 440 445
Trp His Asp Ile Thr Gly Asn Arg Ser Glu Pro Val Val Ile Asn Ser
450 455 460
Glu Gly Trp Gly Glu Phe His Val Asn Gly Gly Ser Val Ser Ile Tyr
465 470 475 480
Val Gln Arg

<210> 35
<211> 511
<212> PRT
<213> Bacillus licheniformis

<400> 35

Met Lys Gln Gln Lys Arg Leu Tyr Ala Arg Leu Leu Thr Leu Leu Phe
1 5 10 15
Ala Leu Ile Phe Leu Leu Pro His Ser Ala Ala Ala Ala Ala Asn Leu
20 25 30
Asn Gly Thr Leu Met Gln Tyr Phe Glu Trp Tyr Met Pro Asn Asp Gly
35 40 45
His Trp Lys Arg Leu Gln Asn Asp Ser Ala Tyr Leu Ala Glu His Gly
50 55 60
Ile Thr Ala Val Trp Ile Pro Pro Ala Tyr Lys Gly Thr Ser Gln Ala
65 70 75 80
Asp Val Gly Tyr Gly Ala Tyr Asp Leu Tyr Asp Leu Gly Glu Phe His
85 90 95
Gln Lys Gly Thr Val Arg Thr Lys Tyr Gly Thr Lys Gly Glu Leu Gln
100 105 110
Ser Ala Ile Lys Ser Leu His Ser Arg Asp Ile Asn Val Tyr Gly Asp
115 120 125
Val Val Ile Asn His Lys Gly Gly Ala Asp Ala Thr Glu Asp Val Thr
130 135 140
Ala Val Glu Val Asp Pro Ala Asp Arg Asn Arg Val Ile Ser Gly Glu
145 150 155 160
His Leu Ile Lys Ala Trp Thr His Phe His Phe Pro Gly Arg Gly Ser
165 170 175
Thr Tyr Ser Asp Phe Lys Trp His Trp Tyr His Phe Asp Gly Thr Asp
180 185 190
Trp Asp Glu Ser Arg Lys Leu Asn Arg Ile Tyr Lys Phe Gln Gly Lys
195 200 205
Ala Trp Asp Trp Glu Val Ser Asn Glu Asn Gly Asn Tyr Asp Tyr Leu
210 215 220
Met Tyr Ala Asp Ile Asp Tyr Asp His Pro Asp Val Ala Ala Glu Ile
225 230 235 240
Lys Arg Trp Gly Thr Trp Tyr Ala Asn Glu Leu Gln Leu Asp Gly Phe
245 250 255
Arg Leu Asp Ala Val Lys His Ile Lys Phe Ser Phe Leu Arg Asp Trp
260 265 270
Val Asn His Val Arg Glu Lys Thr Gly Lys Glu Met Phe Thr Val Ala
275 280 285
Glu Tyr Trp Gln Asn Asp Leu Gly Ala Leu Glu Asn Tyr Leu Asn Lys
290 295 300

```
Thr Asn Phe Asn His Ser Val Phe Asp Val Pro Leu His Tyr Gln Phe
305                 310                 315                 320
His Ala Ala Ser Thr Gln Gly Gly Gly Tyr Asp Met Arg Lys Leu Leu
                325                 330                 335
Asn Gly Thr Val Val Ser Lys His Pro Leu Lys Ser Val Thr Phe Val
            340                 345                 350
Asp Asn His Asp Thr Gln Pro Gly Gln Ser Leu Glu Ser Thr Val Gln
        355                 360                 365
Thr Trp Phe Lys Pro Leu Ala Tyr Ala Phe Ile Leu Thr Arg Glu Ser
    370                 375                 380
Gly Tyr Pro Gln Val Phe Tyr Gly Asp Met Tyr Gly Thr Lys Gly Asp
385                 390                 395                 400
Ser Gln Arg Glu Ile Pro Ala Leu Lys His Lys Ile Glu Pro Ile Leu
                405                 410                 415
Lys Ala Arg Lys Gln Tyr Ala Tyr Gly Ala Gln His Asp Tyr Phe Asp
            420                 425                 430
His His Asp Ile Val Gly Trp Thr Arg Glu Gly Asp Ser Ser Val Ala
        435                 440                 445
Asn Ser Gly Leu Ala Ala Leu Ile Thr Asp Gly Pro Gly Gly Ala Lys
    450                 455                 460
Arg Met Tyr Val Gly Arg Gln Asn Ala Gly Glu Thr Trp His Asp Ile
465                 470                 475                 480
Thr Gly Asn Arg Ser Glu Pro Val Val Ile Asn Ser Glu Gly Trp Gly
                485                 490                 495
Glu Phe His Val Asn Gly Gly Ser Val Ser Ile Tyr Val Gln Arg
            500                 505                 510
```

<210> 36
<211> 520
<212> PRT
<213> Bacillus amyloliquefaciens

<400> 36

```
Met Arg Gly Arg Gly Asn Met Ile Gln Lys Arg Lys Arg Thr Val Ser
 1               5                  10                  15
Phe Arg Leu Val Leu Met Cys Thr Leu Leu Phe Val Ser Leu Pro Ile
            20                  25                  30
Thr Lys Thr Ser Ala Val Asn Gly Thr Leu Met Gln Tyr Phe Glu Trp
        35                  40                  45
Tyr Thr Pro Asn Asp Gly Gln His Trp Lys Arg Leu Gln Asn Asp Ala
    50                  55                  60
Glu His Leu Ser Asp Ile Gly Ile Thr Ala Val Trp Ile Pro Pro Ala
65                  70                  75                  80
Tyr Lys Gly Leu Ser Gln Ser Asp Asn Gly Tyr Gly Pro Tyr Asp Leu
                85                  90                  95
Tyr Asp Leu Gly Glu Phe Gln Gln Lys Gly Thr Val Arg Thr Lys Tyr
            100                 105                 110
Gly Thr Lys Ser Glu Leu Gln Asp Ala Ile Gly Ser Leu His Ser Arg
        115                 120                 125
Asn Val Gln Val Tyr Gly Asp Val Val Leu Asn His Lys Ala Gly Ala
    130                 135                 140
Asp Ala Thr Glu Asp Val Thr Ala Val Glu Val Asn Pro Ala Asn Arg
145                 150                 155                 160
Asn Gln Glu Thr Ser Glu Glu Tyr Gln Ile Lys Ala Trp Thr Asp Phe
                165                 170                 175
Arg Phe Pro Gly Arg Gly Asn Thr Tyr Ser Asp Phe Lys Trp His Trp
            180                 185                 190
Tyr His Phe Asp Gly Ala Asp Trp Asp Glu Ser Arg Lys Ile Ser Arg
        195                 200                 205
```

```
Ile Phe Lys Phe Arg Gly Glu Gly Lys Ala Trp Asp Trp Glu Val Ser
    210                 215                 220
Ser Glu Asn Gly Asn Tyr Asp Tyr Leu Met Tyr Ala Asp Val Asp Tyr
225                 230                 235                 240
Asp His Pro Asp Val Val Ala Glu Thr Lys Lys Trp Gly Ile Trp Tyr
                245                 250                 255
Ala Asn Glu Leu Ser Leu Asp Gly Phe Arg Ile Asp Ala Ala Lys His
            260                 265                 270
Ile Lys Phe Ser Phe Leu Arg Asp Trp Val Gln Ala Val Arg Gln Ala
        275                 280                 285
Thr Gly Lys Glu Met Phe Thr Val Ala Glu Tyr Trp Gln Asn Asn Ala
    290                 295                 300
Gly Lys Leu Glu Asn Tyr Leu Asn Lys Thr Ser Phe Asn Gln Ser Val
305                 310                 315                 320
Phe Asp Val Pro Leu His Phe Asn Leu Gln Ala Ala Ser Ser Gln Gly
                325                 330                 335
Gly Gly Tyr Asp Met Arg Arg Leu Leu Asp Gly Thr Val Val Ser Arg
            340                 345                 350
His Pro Glu Lys Ala Val Thr Phe Val Glu Asn His Asp Thr Gln Pro
        355                 360                 365
Gly Gln Ser Leu Glu Ser Thr Val Gln Thr Trp Phe Lys Pro Leu Ala
    370                 375                 380
Tyr Ala Phe Ile Leu Thr Arg Glu Ser Gly Tyr Pro Gln Val Phe Tyr
385                 390                 395                 400
Gly Asp Met Tyr Gly Thr Lys Gly Thr Ser Pro Lys Glu Ile Pro Ser
                405                 410                 415
Leu Lys Asp Asn Ile Glu Pro Ile Leu Lys Ala Arg Lys Glu Tyr Ala
            420                 425                 430
Tyr Gly Pro Gln His Asp Tyr Ile Asp His Pro Asp Val Ile Gly Trp
        435                 440                 445
Thr Arg Glu Gly Asp Ser Ser Ala Ala Lys Ser Gly Leu Ala Ala Leu
    450                 455                 460
Ile Thr Asp Gly Pro Gly Gly Ser Lys Arg Met Tyr Ala Gly Leu Lys
465                 470                 475                 480
Asn Ala Gly Glu Thr Trp Tyr Asp Ile Thr Gly Asn Arg Ser Asp Thr
                485                 490                 495
Val Lys Ile Gly Ser Asp Gly Trp Gly Glu Phe His Val Asn Asp Gly
            500                 505                 510
Ser Val Ser Ile Tyr Val Gln Lys
        515                 520
```

<210> 37
<211> 548
<212> PRT
<213> Bacillus stearothermophilus

<400> 37

```
Val Leu Thr Phe His Arg Ile Ile Arg Lys Gly Trp Met Phe Leu Leu
1               5                   10                  15
Ala Phe Leu Leu Thr Ala Ser Leu Phe Cys Pro Thr Gly Arg His Ala
            20                  25                  30
Lys Ala Ala Ala Pro Phe Asn Gly Thr Met Met Gln Tyr Phe Glu Trp
        35                  40                  45
Tyr Leu Pro Asp Asp Gly Thr Leu Trp Thr Lys Val Ala Asn Glu Ala
    50                  55                  60
Asn Asn Leu Ser Ser Leu Gly Ile Thr Ala Leu Ser Leu Pro Pro Ala
65                  70                  75                  80
Tyr Lys Gly Thr Ser Arg Ser Asp Val Gly Tyr Gly Val Tyr Asp Leu
                85                  90                  95
```

```
Tyr Asp Leu Gly Glu Phe Asn Gln Lys Gly Thr Val Arg Thr Lys Tyr
            100                 105                 110
Gly Thr Lys Ala Gln Tyr Leu Gln Ala Ile Gln Ala Ala His Ala Ala
        115                 120                 125
Gly Met Gln Val Tyr Ala Asp Val Val Phe Asp His Lys Gly Gly Ala
    130                 135                 140
Asp Gly Thr Glu Trp Val Asp Ala Val Glu Val Asn Pro Ser Asp Arg
145                 150                 155                 160
Asn Gln Glu Ile Ser Gly Thr Tyr Gln Ile Gln Ala Trp Thr Lys Phe
                165                 170                 175
Asp Phe Pro Gly Arg Gly Asn Thr Tyr Ser Ser Phe Lys Trp Arg Trp
            180                 185                 190
Tyr His Phe Asp Gly Val Asp Trp Asp Glu Ser Arg Lys Leu Ser Arg
        195                 200                 205
Ile Tyr Lys Phe Arg Gly Ile Gly Lys Ala Trp Asp Trp Glu Val Asp
    210                 215                 220
Thr Glu Asn Gly Asn Tyr Asp Tyr Leu Met Tyr Ala Asp Leu Asp Met
225                 230                 235                 240
Asp His Pro Glu Val Val Thr Glu Leu Lys Asn Trp Gly Lys Trp Tyr
                245                 250                 255
Val Asn Thr Thr Asn Ile Asp Gly Phe Arg Leu Asp Gly Leu Lys His
            260                 265                 270
Ile Lys Phe Ser Phe Phe Pro Asp Trp Leu Ser Tyr Val Arg Ser Gln
        275                 280                 285
Thr Gly Lys Pro Leu Phe Thr Val Gly Glu Tyr Trp Ser Tyr Asp Ile
    290                 295                 300
Asn Lys Leu His Asn Tyr Ile Thr Lys Thr Asn Gly Thr Met Ser Leu
305                 310                 315                 320
Phe Asp Ala Pro Leu His Asn Lys Phe Tyr Thr Ala Ser Lys Ser Gly
                325                 330                 335
Gly Ala Phe Asp Met Arg Thr Leu Met Thr Asn Thr Leu Met Lys Asp
            340                 345                 350
Gln Pro Thr Leu Ala Val Thr Phe Val Asp Asn His Asp Thr Asn Pro
        355                 360                 365
Ala Lys Arg Cys Ser His Gly Arg Pro Trp Phe Lys Pro Leu Ala Tyr
    370                 375                 380
Ala Phe Ile Leu Thr Arg Gln Glu Gly Tyr Pro Cys Val Phe Tyr Gly
385                 390                 395                 400
Asp Tyr Tyr Gly Ile Pro Gln Tyr Asn Ile Pro Ser Leu Lys Ser Lys
                405                 410                 415
Ile Asp Pro Leu Leu Ile Ala Arg Arg Asp Tyr Ala Tyr Gly Thr Gln
            420                 425                 430
His Asp Tyr Leu Asp His Ser Asp Ile Ile Gly Trp Thr Arg Glu Gly
        435                 440                 445
Val Thr Glu Lys Pro Gly Ser Gly Leu Ala Ala Leu Ile Thr Asp Gly
    450                 455                 460
Ala Gly Arg Ser Lys Trp Met Tyr Val Gly Lys Gln His Ala Gly Lys
465                 470                 475                 480
Val Phe Tyr Asp Leu Thr Gly Asn Arg Ser Asp Thr Val Thr Ile Asn
                485                 490                 495
Ser Asp Gly Trp Gly Glu Phe Lys Val Asn Gly Gly Ser Val Ser Val
            500                 505                 510
Trp Val Pro Arg Lys Thr Thr Val Ser Thr Ile Ala Arg Pro Ile Thr
        515                 520                 525
Thr Arg Pro Trp Thr Gly Glu Phe Val Arg Trp His Glu Pro Arg Leu
    530                 535                 540
Val Ala Trp Pro
545
```

<210> 38
<211> 32
<212> PRT
<213> Bacillus licheniformis

<400> 38

```
Met Lys Gln Gln Lys Arg Leu Thr Ala Arg Leu Leu Thr Leu Leu Phe
 1               5                  10                  15
Ala Leu Ile Phe Leu Leu Pro His Ser Ala Ala Ala Ala Ala Asn Leu
            20                  25                  30
```

<210> 39
<211> 33
<212> PRT
<213> Bacillus subtilis

<400> 39

```
Met Arg Ser Lys Thr Leu Trp Ile Ser Leu Leu Phe Ala Leu Thr Leu
 1               5                  10                  15
Ile Phe Thr Met Ala Phe Ser Asn Met Ser Ala Gln Ala Ala Gly Lys
            20                  25                  30
Ser
```

<210> 40
<211> 32
<212> PRT
<213> Bacillus licheniformis

<400> 40

```
Met Arg Ser Lys Thr Leu Trp Ile Ser Leu Leu Phe Ala Leu Thr Leu
 1               5                  10                  15
Ile Phe Thr Met Ala Phe Ser Asn Met Ser Ala Gln Ala Ala Asn Leu
            20                  25                  30
```

<210> 41
<211> 234
<212> PRT
<213> Trichoerma reesei

<400> 41

```
Met Lys Phe Leu Gln Val Leu Pro Ala Leu Ile Pro Ala Ala Leu Ala
 1               5                  10                  15
Gln Thr Ser Cys Asp Gln Trp Ala Thr Phe Thr Gly Asn Gly Tyr Thr
            20                  25                  30
Val Ser Asn Asn Leu Trp Gly Ala Ser Ala Gly Ser Gly Phe Gly Cys
        35                  40                  45
Val Thr Ala Val Ser Leu Ser Gly Gly Ala Ser Trp His Ala Asp Trp
    50                  55                  60
Gln Trp Ser Gly Gly Gln Asn Asn Val Lys Ser Tyr Gln Asn Ser Gln
65                  70                  75                  80
Ile Ala Ile Pro Gln Lys Arg Thr Val Asn Ser Ile Ser Ser Met Pro
                85                  90                  95
Thr Thr Ala Ser Trp Ser Tyr Ser Gly Ser Asn Ile Arg Ala Asn Val
            100                 105                 110
Ala Tyr Asp Leu Phe Thr Ala Ala Asn Pro Asn His Val Thr Tyr Ser
        115                 120                 125
Gly Asp Tyr Glu Leu Met Ile Trp Leu Gly Lys Tyr Gly Asp Ile Gly
    130                 135                 140
```

```
Pro Ile Gly Ser Ser Gln Gly Thr Val Asn Val Gly Gly Gln Ser Trp
145                 150                 155                 160
Thr Leu Tyr Tyr Gly Tyr Asn Gly Ala Met Gln Val Tyr Ser Phe Val
                165                 170                 175
Ala Gln Thr Asn Thr Thr Asn Tyr Ser Gly Asp Val Lys Asn Phe Phe
            180                 185                 190
Asn Tyr Leu Arg Asp Asn Lys Gly Tyr Asn Ala Ala Gly Gln Tyr Val
        195                 200                 205
Leu Ser Tyr Gln Phe Gly Thr Glu Pro Phe Thr Gly Ser Gly Thr Leu
    210                 215                 220
Asn Val Ala Ser Trp Thr Ala Ser Ile Asn
225                 230
```

<210> 42
<211> 234
<212> PRT
<213> Hypocrea schweinitzii

<400> 42

```
Met Lys Phe Leu Gln Val Leu Pro Ala Ile Leu Pro Ala Ala Leu Ala
 1               5                  10                  15
Gln Thr Ser Cys Asp Gln Tyr Ala Thr Phe Ser Gly Asn Gly Tyr Ile
            20                  25                  30
Val Ser Asn Asn Leu Trp Gly Ala Ser Ala Gly Ser Gly Phe Gly Cys
        35                  40                  45
Val Thr Ser Val Ser Leu Asn Gly Ala Ala Ser Trp His Ala Asp Trp
    50                  55                  60
Gln Trp Ser Gly Gly Gln Asn Asn Val Lys Ser Tyr Gln Asn Val Gln
65                  70                  75                  80
Ile Asn Ile Pro Gln Lys Arg Thr Val Asn Ser Ile Gly Ser Met Pro
                85                  90                  95
Thr Thr Ala Ser Trp Ser Tyr Ser Gly Ser Asp Ile Arg Ala Asn Val
            100                 105                 110
Ala Tyr Asp Leu Phe Thr Ala Ala Asn Pro Asn His Val Thr Tyr Ser
        115                 120                 125
Gly Asp Tyr Glu Leu Met Ile Trp Leu Gly Lys Tyr Gly Asp Ile Gly
    130                 135                 140
Pro Ile Gly Ser Ser Gln Gly Thr Val Asn Val Gly Gly Gln Thr Trp
145                 150                 155                 160
Thr Leu Tyr Tyr Gly Tyr Asn Gly Ala Met Gln Val Tyr Ser Phe Val
                165                 170                 175
Ala Gln Ser Asn Thr Thr Ser Tyr Ser Gly Asp Val Lys Asn Phe Phe
            180                 185                 190
Asn Tyr Leu Arg Asp Asn Lys Gly Tyr Asn Ala Gly Gly Gln Tyr Val
        195                 200                 205
Leu Ser Tyr Gln Phe Gly Thr Glu Pro Phe Thr Gly Ser Gly Thr Leu
    210                 215                 220
Asn Val Ala Ser Trp Thr Ala Ser Ile Asn
225                 230
```

<210> 43
<211> 258
<212> PRT
<213> Aspergillus aculeatus

<400> 43

```
Met Lys Ala Phe His Leu Leu Ala Ala Leu Ala Gly Ala Ala Val Ala
 1               5                  10                  15
Gln Gln Ala Gln Leu Cys Asp Gln Tyr Ala Thr Tyr Thr Gly Gly Val
```

```
                20                  25                  30
Tyr Thr Ile Asn Asn Asn Leu Trp Gly Lys Asp Ala Gly Ser Gly Ser
        35                  40                  45
Gln Cys Thr Thr Val Asn Ser Ala Ser Ser Ala Gly Thr Ser Trp Ser
    50                  55                  60
Thr Lys Trp Asn Trp Ser Gly Gly Glu Asn Ser Val Lys Ser Tyr Ala
65                  70                  75                  80
Asn Ser Gly Leu Thr Phe Asn Lys Lys Leu Val Ser Gln Ile Ser Gln
                85                  90                  95
Ile Pro Thr Thr Ala Arg Trp Ser Tyr Asp Asn Thr Gly Ile Arg Ala
            100                 105                 110
Asp Val Ala Tyr Asp Leu Phe Thr Ala Ala Asp Ile Asn His Val Thr
        115                 120                 125
Trp Ser Gly Asp Tyr Glu Leu Met Ile Trp Leu Ala Arg Tyr Gly Gly
    130                 135                 140
Val Gln Pro Ile Gly Ser Gln Ile Ala Thr Ala Thr Val Asp Gly Gln
145                 150                 155                 160
Thr Trp Glu Leu Trp Tyr Gly Ala Asn Gly Ser Gln Lys Thr Tyr Ser
                165                 170                 175
Phe Val Ala Pro Thr Pro Ile Thr Ser Phe Gln Gly Asp Val Asn Asp
            180                 185                 190
Phe Phe Lys Tyr Leu Thr Gln Asn His Gly Phe Pro Ala Ser Ser Gln
        195                 200                 205
Tyr Leu Ile Thr Leu Gln Phe Gly Thr Glu Pro Phe Thr Gly Gly Pro
    210                 215                 220
Ala Thr Leu Ser Val Ser Asn Trp Ser Ala Ser Val Gln Gln Ala Gly
225                 230                 235                 240
Glu Pro Trp Gln Asn Gly Ala Gly Leu Ala Val Asn Ser Phe Ser Ser
                245                 250                 255
Thr Val
```

<210> 44
<211> 239
<212> PRT
<213> Aspergillus kawachii

<400> 44

```
Met Lys Ala Phe His Leu Leu Ala Ala Leu Ser Gly Ala Ala Val Ala
 1               5                  10                  15
Gln Gln Ala Gln Leu Cys Asp Gln Tyr Ala Thr Tyr Thr Gly Gly Val
                20                  25                  30
Tyr Thr Ile Asn Asn Asn Leu Trp Gly Lys Asp Ala Gly Ser Gly Ser
        35                  40                  45
Gln Cys Thr Thr Val Asn Ser Ala Ser Ser Ala Gly Thr Ser Trp Ser
    50                  55                  60
Thr Lys Trp Asn Trp Ser Gly Gly Glu Asn Ser Val Lys Ser Tyr Ala
65                  70                  75                  80
Asn Ser Gly Leu Ser Phe Asn Lys Lys Leu Val Ser Gln Ile Ser His
                85                  90                  95
Ile Pro Thr Ala Ala Arg Trp Ser Tyr Asp Asn Thr Cys Ile Arg Arg
            100                 105                 110
Gly Arg Ala Tyr Asp Leu Phe Thr Ala Ala Asp Ile Asn His Val Thr
        115                 120                 125
Trp Ser Gly Asp Tyr Glu Leu Met Ile Trp Leu Ala Arg Tyr Gly Gly
    130                 135                 140
Val Gln Pro Leu Gly Ser Gln Ile Ala Thr Ala Thr Val Glu Gly Gln
145                 150                 155                 160
Thr Trp Glu Leu Trp Tyr Gly Val Asn Gly Ala Gln Lys Thr Tyr Ser
```

```
                165                 170                 175
Phe Val Ala Ala Asn Pro Ile Thr Ser Phe Gln Gly Asp Ile Asn Asp
            180                 185                 190
Phe Phe Lys Tyr Leu Thr Gln Asn His Gly Phe Pro Ala Ser Ser Gln
        195                 200                 205
Tyr Leu Ile Ile Leu Ala Leu Gln Phe Gly Thr Glu Pro Phe Thr Gly
    210                 215                 220
Gly Pro Ala Thr Leu Asn Val Ala Asp Trp Ser Ala Ser Val Gln
225                 230                 235
```

<210> 45
<211> 254
<212> PRT
<213> Humicola grisea

<400> 45

```
Met Leu Lys Ser Ala Leu Leu Leu Gly Ala Ala Ala Val Ser Val Gln
 1               5                  10                  15
Ser Ala Ser Ile Pro Thr Ile Pro Ala Asn Leu Glu Pro Arg Gln Ile
            20                  25                  30
Arg Ser Leu Cys Glu Leu Tyr Gly Tyr Trp Ser Gly Asn Gly Tyr Glu
        35                  40                  45
Leu Leu Asn Asn Leu Trp Gly Lys Asp Thr Ala Thr Ser Gly Trp Gln
    50                  55                  60
Cys Thr Tyr Leu Asp Gly Thr Asn Asn Gly Gly Ile Gln Trp Asn Thr
65                  70                  75                  80
Ala Trp Glu Trp Gln Gly Ala Pro Asp Asn Val Lys Asn Tyr Pro Tyr
                85                  90                  95
Val Gly Lys Gln Ile Gln Arg Gly Arg Lys Ile Ser Asp Ile Asn Ser
            100                 105                 110
Met Arg Thr Ser Val Ser Trp Thr Tyr Asp Arg Thr Asp Leu Arg Ala
        115                 120                 125
Asn Val Ala Tyr Asp Val Phe Thr Ala Arg Asp Pro Asp His Pro Asn
    130                 135                 140
Trp Gly Gly Asp Tyr Glu Leu Met Ile Trp Leu Ala Arg Tyr Gly Gly
145                 150                 155                 160
Ile Tyr Pro Ile Gly Thr Phe His Ser Gln Val Asn Leu Ala Gly Arg
                165                 170                 175
Thr Trp Asp Leu Trp Thr Gly Tyr Asn Gly Asn Met Arg Val Tyr Ser
            180                 185                 190
Phe Leu Pro Pro Ser Gly Asp Ile Arg Asp Phe Ser Cys Asp Ile Lys
        195                 200                 205
Asp Phe Phe Asn Tyr Leu Glu Arg Asn His Gly Tyr Pro Ala Arg Glu
    210                 215                 220
Gln Asn Leu Ile Val Tyr Gln Val Gly Thr Glu Cys Phe Thr Gly Gly
225                 230                 235                 240
Pro Ala Arg Phe Thr Cys Arg Asp Phe Arg Ala Asp Leu Trp
                245                 250
```

<210> 46
<211> 238
<212> PRT
<213> Gliocladium roseum (1)

<400> 46

```
Met Lys Ala Asn Ile Val Ile Leu Ser Leu Phe Ala Pro Leu Ala Ala
 1               5                  10                  15
Val Ala Gln Thr Leu Cys Gly Gln Tyr Ser Ser Asn Thr Gln Gly Gly
            20                  25                  30
```

```
Tyr Ile Phe Asn Asn Asn Met Trp Gly Met Gly Ser Gly Ser Gly Ser
        35                  40                  45
Gln Cys Thr Tyr Val Asp Lys Val Trp Ala Glu Gly Val Ala Trp His
    50                  55                  60
Thr Asp Trp Ser Trp Ser Gly Gly Asp Asn Asn Val Lys Ser Tyr Pro
65                  70                  75                  80
Tyr Ser Gly Arg Glu Leu Gly Thr Lys Arg Ile Val Ser Ser Ile Lys
                85                  90                  95
Ser Ile Ser Ser Gly Ala Asp Trp Asp Tyr Thr Gly Ser Asn Leu Arg
            100                 105                 110
Ala Asn Ala Ala Tyr Asp Ile Phe Thr Ser Ala Asn Pro Asn His Ala
        115                 120                 125
Thr Ser Ser Gly Asp Tyr Glu Val Met Ile Trp Leu Ala Asn Leu Gly
    130                 135                 140
Gly Leu Thr Pro Ile Gly Ser Pro Ile Gly Thr Val Lys Ala Ala Gly
145                 150                 155                 160
Arg Asp Trp Glu Leu Trp Asp Gly Tyr Asn Gly Ala Met Arg Val Tyr
                165                 170                 175
Ser Phe Val Ala Pro Ser Gln Leu Asn Ser Phe Asp Gly Glu Ile Met
            180                 185                 190
Asp Phe Phe Tyr Val Val Lys Asp Met Arg Gly Phe Pro Ala Asp Ser
        195                 200                 205
Gln His Leu Leu Thr Val Gln Phe Gly Thr Glu Pro Ile Ser Gly Ser
    210                 215                 220
Gly Ala Lys Phe Ser Val Ser His Trp Ser Ala Lys Leu Gly
225                 230                 235
```

<210> 47
<211> 236
<212> PRT
<213> Gliocladium roesum (2)

<400> 47

```
Met Lys Phe Gln Leu Leu Ser Leu Thr Ala Phe Ala Pro Leu Ser Leu
 1              5                   10                  15
Ala Ala Leu Cys Gly Gln Tyr Gln Ser Gln Ser Gln Gly Gly Tyr Ile
            20                  25                  30
Phe Asn Asn Asn Lys Trp Gly Gln Gly Ser Gly Ser Gly Ser Gln Cys
        35                  40                  45
Leu Thr Ile Asp Lys Thr Trp Asp Ser Asn Val Ala Phe His Ala Asp
    50                  55                  60
Trp Ser Trp Ser Gly Gly Thr Asn Asn Val Lys Ser Tyr Pro Asn Ala
65                  70                  75                  80
Gly Leu Glu Phe Ser Arg Gly Lys Lys Val Ser Ser Ile Gly Thr Ile
                85                  90                  95
Asn Gly Gly Ala Asp Trp Asp Tyr Ser Gly Ser Asn Ile Arg Ala Asn
            100                 105                 110
Val Ala Tyr Gly Ile Phe Thr Ser Ala Asp Pro Asn His Val Thr Ser
        115                 120                 125
Ser Gly Asp Tyr Glu Leu Met Ile Trp Leu Gly Lys Leu Gly Asp Ile
    130                 135                 140
Tyr Pro Ile Gly Asn Ser Ile Gly Arg Val Glu Ala Ala Asn Arg Glu
145                 150                 155                 160
Trp Asp Phe Leu Val Gly Tyr Asn Gly Ala Met Lys Val Phe Ser Phe
                165                 170                 175
Val Ala Pro Ser Pro Val Thr Leu Phe Asp Gly Asn Ile Met Asp Phe
            180                 185                 190
Phe Tyr Val Met Arg Asp Met Gln Gly Tyr Pro Met Asp Lys Gln Tyr
        195                 200                 205
```

```
Leu Leu Ser Leu Gln Phe Gly Thr Glu Pro Phe Thr Gly Ser Asn Ala
    210                 215                 220
Asn Phe Ser Cys Trp Tyr Phe Gly Ala Lys Ile Lys
225                 230                 235
```

<210> 48
<211> 237
<212> PRT
<213> Gliocladium roseum (3)

<400> 48

```
Met Lys Thr Gly Ile Ala Tyr Leu Ala Ala Val Leu Pro Leu Ala Met
 1              5                   10                  15
Ala Glu Ser Leu Cys Asp Gln Tyr Ala Tyr Leu Ser Arg Asp Gly Tyr
            20                  25                  30
Asn Phe Asn Asn Asn Glu Trp Gly Ala Ala Thr Gly Thr Gly Asp Gln
    35                  40                  45
Cys Thr Tyr Val Asp Ser Thr Ser Ser Gly Gly Val Ser Trp His Ser
    50                  55                  60
Asp Trp Thr Trp Ser Gly Ser Glu Ser Glu Ile Lys Ser Tyr Pro Tyr
65                  70                  75                  80
Ser Gly Leu Asp Leu Pro Glu Lys Lys Ile Val Thr Ser Ile Gly Ser
                85                  90                  95
Ile Ser Thr Gly Ala Glu Trp Ser Tyr Ser Gly Ser Asp Ile Arg Ala
            100                 105                 110
Asp Val Ala Tyr Asp Thr Phe Thr Ala Ala Asp Pro Asn His Ala Thr
        115                 120                 125
Ser Ser Gly Asp Tyr Glu Val Met Ile Trp Leu Ala Asn Leu Gly Gly
    130                 135                 140
Leu Thr Pro Ile Gly Ser Pro Ile Gly Thr Val Lys Ala Ala Gly Arg
145                 150                 155                 160
Asp Trp Glu Leu Trp Asp Gly Tyr Asn Gly Ala Met Arg Val Tyr Ser
                165                 170                 175
Phe Val Ala Pro Ser Gln Leu Asn Ser Phe Asp Gly Glu Ile Met Asp
            180                 185                 190
Phe Phe Tyr Val Val Lys Asp Met Arg Gly Phe Pro Ala Asp Ser Gln
        195                 200                 205
His Leu Leu Thr Val Gln Phe Gly Thr Glu Pro Ile Ser Gly Ser Gly
    210                 215                 220
Ala Lys Phe Ser Val Ser His Trp Ser Ala Lys Leu Gly
225                 230                 235
```